# EUROPEAN PATENT APPLICATION

(11) **EP 1 880 728 A1**
(43) Date of publication of application: **23.01.2008**
(21) Application number: 06745679.8
(22) Date of filing: 26.04.2006
(51) Int. Cl.: A61K 38/00

(54) **PEPTIDE HAVING ANTI-ANXIETY EFFECT AND METHOD FOR SCREENING THEREOF**

(30) Priority: 26.04.2005 JP 2005128140
(71) Applicant: Eisai R&D Management Co., Ltd., Tokyo 112-8088 (JP)
(72) Inventor: HIDA, Takayuki c/o Eisai Co., Ltd., Ibaraki 3002635 (JP); ARAI, Turu c/o Eisai Co., Ltd., Ibaraki 3002635 (JP); SEKIYA, Tomoko c/o Eisai Co., Ltd., Ibaraki 3002635 (JP); TAKAHASHI, Eiki c/o Eisai Co., Ltd., Ibaraki 3002635 (JP); SHIKATA, Kodo c/o Eisai Co., Ltd., Ibaraki 3002635 (JP)
(74) Representative: Gillard, Richard Edward
(86) International application number: PCT/JP2006/308701
(87) International publication number: WO 2006/118131

(57) **Abstract**

The objects of the present invention are to provide a polypeptide having an antianxiety activity; a therapeutic agent containing the polypeptide; a method for treating anxiety using the polypeptide; a method of screening for a compound capable of activating or suppressing a receptor for the polypeptide and involved in the regulation of anxiety, a salt thereof, or a hydrate of them; and a kit for the screening. There is provided an antianxiety agent containing relaxin-3.

SEQUENCE LISTING

<110> Eisai R&D Management Co., Ltd.

<120> Peptides having antianxiety activity and screening methods

<130> 158444PX

<160> 20

<170> PatentIn version 3.1

<210> 1
<211> 429
<212> DNA
<213> Homo sapiens

<220>
<221> CDS
<222> (1)..(429)
<223>

<400> 1
atg gcc agg tac atg ctg ctg ctg ctc ctg gcg gta tgg gtg ctg acc 48
Met Ala Arg Tyr Met Leu Leu Leu Leu Leu Ala Val Trp Val Leu Thr
1               5                   10                  15

ggg gag ctg tgg ccg gga gct gag gcc cgg gca gcg cct tac ggg gtc 96
Gly Glu Leu Trp Pro Gly Ala Glu Ala Arg Ala Ala Pro Tyr Gly Val
            20                  25                  30

agg ctt tgc ggc cga gaa ttc atc cga gca gtc atc ttc acc tgc ggg 144
Arg Leu Cys Gly Arg Glu Phe Ile Arg Ala Val Ile Phe Thr Cys Gly
        35                  40                  45

ggc tcc cgg tgg aga cga tca gac atc ctg gcc cac gag gct atg gga 192
Gly Ser Arg Trp Arg Arg Ser Asp Ile Leu Ala His Glu Ala Met Gly
    50                  55                  60

gat acc ttc ccg gat gca gat gct gat gaa gac agt ctg gca ggc gag 240
Asp Thr Phe Pro Asp Ala Asp Ala Asp Glu Asp Ser Leu Ala Gly Glu
65                  70                  75                  80

ctg gat gag gcc atg ggg tcc agc gag tgg ctg gcc ctg acc aag tca 288
Leu Asp Glu Ala Met Gly Ser Ser Glu Trp Leu Ala Leu Thr Lys Ser
                85                  90                  95

ccc cag gcc ttt tac agg ggg cga ccc agc tgg caa gga acc cct ggg 336

Pro Gln Ala Phe Tyr Arg Gly Arg Pro Ser Trp Gln Gly Thr Pro Gly
            100                 105                 110

gtt ctt cgg ggc agc cga gat gtc ctg gct ggc ctt tcc agc agc tgc 384
Val Leu Arg Gly Ser Arg Asp Val Leu Ala Gly Leu Ser Ser Ser Cys
        115                 120                 125

tgc aag tgg ggg tgt agc aaa agt gaa atc agt agc ctt tgc tag 429
Cys Lys Trp Gly Cys Ser Lys Ser Glu Ile Ser Ser Leu Cys
    130                 135                 140

<210> 2
<211> 142
<212> PRT
<213> Homo sapiens

<400> 2

Met Ala Arg Tyr Met Leu Leu Leu Leu Leu Ala Val Trp Val Leu Thr
1               5                   10                  15

Gly Glu Leu Trp Pro Gly Ala Glu Ala Arg Ala Ala Pro Tyr Gly Val
            20                  25                  30

Arg Leu Cys Gly Arg Glu Phe Ile Arg Ala Val Ile Phe Thr Cys Gly
        35                  40                  45

Gly Ser Arg Trp Arg Arg Ser Asp Ile Leu Ala His Glu Ala Met Gly
    50                  55                  60

Asp Thr Phe Pro Asp Ala Asp Ala Asp Glu Asp Ser Leu Ala Gly Glu
65                  70                  75                  80

Leu Asp Glu Ala Met Gly Ser Ser Glu Trp Leu Ala Leu Thr Lys Ser
                85                  90                  95

Pro Gln Ala Phe Tyr Arg Gly Arg Pro Ser Trp Gln Gly Thr Pro Gly
            100                 105                 110

Val Leu Arg Gly Ser Arg Asp Val Leu Ala Gly Leu Ser Ser Ser Cys

115                 120                 125

Cys Lys Trp Gly Cys Ser Lys Ser Glu Ile Ser Ser Leu Cys
    130                 135                 140

<210>  3
<211>  1410
<212>  DNA
<213>  Homo sapiens

<220>
<221>  CDS
<222>  (1)..(1410)
<223>

<400>  3
atg cag atg gcc gat gca gcc acg ata gcc acc atg aat aag gca gca      48
Met Gln Met Ala Asp Ala Ala Thr Ile Ala Thr Met Asn Lys Ala Ala
1               5                   10                  15

ggc ggg gac aag cta gca gaa ctc ttc agt ctg gtc ccg gac ctt ctg      96
Gly Gly Asp Lys Leu Ala Glu Leu Phe Ser Leu Val Pro Asp Leu Leu
            20                  25                  30

gag gcg gcc aac acg agt ggt aac gcg tcg ctg cag ctt ccg gac ttg      144
Glu Ala Ala Asn Thr Ser Gly Asn Ala Ser Leu Gln Leu Pro Asp Leu
        35                  40                  45

tgg tgg gag ctg ggg ctg gag ttg ccg gac ggc gcg ccg cca gga cat      192
Trp Trp Glu Leu Gly Leu Glu Leu Pro Asp Gly Ala Pro Pro Gly His
    50                  55                  60

ccc ccg ggc agc ggc ggg gca gag agc gcg gac aca gag gcc cgg gtg      240
Pro Pro Gly Ser Gly Gly Ala Glu Ser Ala Asp Thr Glu Ala Arg Val
65                  70                  75                  80

cgg att ctc atc agc gtg gtg tac tgg gtg gtg tgc gcc ctg ggg ttg      288
Arg Ile Leu Ile Ser Val Val Tyr Trp Val Val Cys Ala Leu Gly Leu
                85                  90                  95

gcg ggc aac ctg ctg gtt ctc tac ctg atg aag agc atg cag ggc tgg      336
Ala Gly Asn Leu Leu Val Leu Tyr Leu Met Lys Ser Met Gln Gly Trp
            100                 105                 110

cgc aag tcc tct atc aac ctc ttc gtc acc aac ctg gcg ctg acg gac      384
Arg Lys Ser Ser Ile Asn Leu Phe Val Thr Asn Leu Ala Leu Thr Asp

        115                 120                 125

ttt cag ttt gtg ctc acc ctg ccc ttc tgg gcg gtg gag aac gct ctt 432
Phe Gln Phe Val Leu Thr Leu Pro Phe Trp Ala Val Glu Asn Ala Leu
    130                 135                 140

gac ttc aaa tgg ccc ttc ggc aag gcc atg tgt aag atc gtg tcc atg 480
Asp Phe Lys Trp Pro Phe Gly Lys Ala Met Cys Lys Ile Val Ser Met
145                 150                 155                 160

gtg acg tcc atg aac atg tac gcc agc gtg ttc ttc ctc act gcc atg 528
Val Thr Ser Met Asn Met Tyr Ala Ser Val Phe Phe Leu Thr Ala Met
                165                 170                 175

agt gtg acg cgc tac cat tcg gtg gcc tcg gct ctg aag agc cac cgg 576
Ser Val Thr Arg Tyr His Ser Val Ala Ser Ala Leu Lys Ser His Arg
            180                 185                 190

acc cga gga cac ggc cgg ggc gac tgc tgc ggc cgg agc ctg ggg gac 624
Thr Arg Gly His Gly Arg Gly Asp Cys Cys Gly Arg Ser Leu Gly Asp
        195                 200                 205

agc tgc tgc ttc tcg gcc aag gcg ctg tgt gtg tgg atc tgg gct ttg 672
Ser Cys Cys Phe Ser Ala Lys Ala Leu Cys Val Trp Ile Trp Ala Leu
    210                 215                 220

gcc gcg ctg gcc tcg ctg ccc agt gcc att ttc tcc acc acg gtc aag 720
Ala Ala Leu Ala Ser Leu Pro Ser Ala Ile Phe Ser Thr Thr Val Lys
225                 230                 235                 240

gtg atg ggc gag gag ctg tgc ctg gtg cgt ttc ccg gac aag ttg ctg 768
Val Met Gly Glu Glu Leu Cys Leu Val Arg Phe Pro Asp Lys Leu Leu
                245                 250                 255

ggc cgc gac agg cag ttc tgg ctg ggc ctc tac cac tcg cag aag gtg 816
Gly Arg Asp Arg Gln Phe Trp Leu Gly Leu Tyr His Ser Gln Lys Val
            260                 265                 270

ctg ttg ggc ttc gtg ctg ccg ctg ggc atc att atc ttg tgc tac ctg 864
Leu Leu Gly Phe Val Leu Pro Leu Gly Ile Ile Ile Leu Cys Tyr Leu
        275                 280                 285

ctg ctg gtg cgc ttc atc gcc gac cgc cgc gcg gcg ggg acc aaa gga 912
Leu Leu Val Arg Phe Ile Ala Asp Arg Arg Ala Ala Gly Thr Lys Gly
    290                 295                 300

ggg gcc gcg gta gcc gga gga cgc ccg acc gga gcc agc gcc cgg aga 960
Gly Ala Ala Val Ala Gly Gly Arg Pro Thr Gly Ala Ser Ala Arg Arg

305                 310                 315                 320

ctg tcg aag gtc acc aaa tca gtg acc atc gtt gtc ctg tcc ttc ttc      1008
Leu Ser Lys Val Thr Lys Ser Val Thr Ile Val Val Leu Ser Phe Phe
                325                 330                 335

ctg tgt tgg ctg ccc aac cag gcg ctc acc acc tgg agc atc ctc atc      1056
Leu Cys Trp Leu Pro Asn Gln Ala Leu Thr Thr Trp Ser Ile Leu Ile
            340                 345                 350

aag ttc aac gcg gtg ccc ttc agc cag gag tat ttc ctg tgc cag gta      1104
Lys Phe Asn Ala Val Pro Phe Ser Gln Glu Tyr Phe Leu Cys Gln Val
        355                 360                 365

tac gcg ttc cct gtg agc gtg tgc cta gcg cac tcc aac agc tgc ctc      1152
Tyr Ala Phe Pro Val Ser Val Cys Leu Ala His Ser Asn Ser Cys Leu
    370                 375                 380

aac ccc gtc ctc tac tgc ctc gtg cgc cgc gag ttc cgc aag gcg ctc      1200
Asn Pro Val Leu Tyr Cys Leu Val Arg Arg Glu Phe Arg Lys Ala Leu
385                 390                 395                 400

aag agc ctg ctg tgg cgc atc gcg tct cct tcg atc acc agc atg cgc      1248
Lys Ser Leu Leu Trp Arg Ile Ala Ser Pro Ser Ile Thr Ser Met Arg
                405                 410                 415

ccc ttc acc gcc act acc aag ccg gag cac gag gat cag ggg ctg cag      1296
Pro Phe Thr Ala Thr Thr Lys Pro Glu His Glu Asp Gln Gly Leu Gln
            420                 425                 430

gcc ccg gcg ccg ccc cac gcg gcc gcg gag ccg gac ctg ctc tac tac      1344
Ala Pro Ala Pro Pro His Ala Ala Ala Glu Pro Asp Leu Leu Tyr Tyr
        435                 440                 445

cca cct ggc gtc gtg gtc tac agc ggg ggg cgc tac gac ctg ctg ccc      1392
Pro Pro Gly Val Val Val Tyr Ser Gly Gly Arg Tyr Asp Leu Leu Pro
    450                 455                 460

agc agc tct gcc tac tga                                              1410
Ser Ser Ser Ala Tyr
465


<210>  4
<211>  469
<212>  PRT
<213>  Homo sapiens

<400> 4

Met Gln Met Ala Asp Ala Ala Thr Ile Ala Thr Met Asn Lys Ala Ala
1               5                   10                  15

Gly Gly Asp Lys Leu Ala Glu Leu Phe Ser Leu Val Pro Asp Leu Leu
            20                  25                  30

Glu Ala Ala Asn Thr Ser Gly Asn Ala Ser Leu Gln Leu Pro Asp Leu
        35                  40                  45

Trp Trp Glu Leu Gly Leu Glu Leu Pro Asp Gly Ala Pro Pro Gly His
    50                  55                  60

Pro Pro Gly Ser Gly Gly Ala Glu Ser Ala Asp Thr Glu Ala Arg Val
65                  70                  75                  80

Arg Ile Leu Ile Ser Val Val Tyr Trp Val Val Cys Ala Leu Gly Leu
                85                  90                  95

Ala Gly Asn Leu Leu Val Leu Tyr Leu Met Lys Ser Met Gln Gly Trp
            100                 105                 110

Arg Lys Ser Ser Ile Asn Leu Phe Val Thr Asn Leu Ala Leu Thr Asp
        115                 120                 125

Phe Gln Phe Val Leu Thr Leu Pro Phe Trp Ala Val Glu Asn Ala Leu
    130                 135                 140

Asp Phe Lys Trp Pro Phe Gly Lys Ala Met Cys Lys Ile Val Ser Met
145                 150                 155                 160

Val Thr Ser Met Asn Met Tyr Ala Ser Val Phe Phe Leu Thr Ala Met
                165                 170                 175

Ser Val Thr Arg Tyr His Ser Val Ala Ser Ala Leu Lys Ser His Arg
            180                 185                 190

Thr Arg Gly His Gly Arg Gly Asp Cys Cys Gly Arg Ser Leu Gly Asp
        195                 200                 205

Ser Cys Cys Phe Ser Ala Lys Ala Leu Cys Val Trp Ile Trp Ala Leu
    210                 215                 220

Ala Ala Leu Ala Ser Leu Pro Ser Ala Ile Phe Ser Thr Thr Val Lys
225                 230                 235                 240

Val Met Gly Glu Glu Leu Cys Leu Val Arg Phe Pro Asp Lys Leu Leu
                245                 250                 255

Gly Arg Asp Arg Gln Phe Trp Leu Gly Leu Tyr His Ser Gln Lys Val
            260                 265                 270

Leu Leu Gly Phe Val Leu Pro Leu Gly Ile Ile Ile Leu Cys Tyr Leu
        275                 280                 285

Leu Leu Val Arg Phe Ile Ala Asp Arg Arg Ala Ala Gly Thr Lys Gly
    290                 295                 300

Gly Ala Ala Val Ala Gly Gly Arg Pro Thr Gly Ala Ser Ala Arg Arg
305                 310                 315                 320

Leu Ser Lys Val Thr Lys Ser Val Thr Ile Val Val Leu Ser Phe Phe
                325                 330                 335

Leu Cys Trp Leu Pro Asn Gln Ala Leu Thr Thr Trp Ser Ile Leu Ile
            340                 345                 350

Lys Phe Asn Ala Val Pro Phe Ser Gln Glu Tyr Phe Leu Cys Gln Val
        355                 360                 365

Tyr Ala Phe Pro Val Ser Val Cys Leu Ala His Ser Asn Ser Cys Leu
    370                 375                 380

Asn Pro Val Leu Tyr Cys Leu Val Arg Arg Glu Phe Arg Lys Ala Leu
385                 390                 395                 400

Lys Ser Leu Leu Trp Arg Ile Ala Ser Pro Ser Ile Thr Ser Met Arg
                405                 410                 415

Pro Phe Thr Ala Thr Thr Lys Pro Glu His Glu Asp Gln Gly Leu Gln
            420                 425                 430

Ala Pro Ala Pro Pro His Ala Ala Ala Glu Pro Asp Leu Leu Tyr Tyr
        435                 440                 445

Pro Pro Gly Val Val Val Tyr Ser Gly Gly Arg Tyr Asp Leu Leu Pro
    450                 455                 460

Ser Ser Ser Ala Tyr
465

<210> 5
<211> 27
<212> PRT
<213> Homo sapiens

<400> 5

Arg Ala Ala Pro Tyr Gly Val Arg Leu Cys Gly Arg Glu Phe Ile Arg
1               5                   10                  15

Ala Val Ile Phe Thr Cys Gly Gly Ser Arg Trp
            20                  25

<210> 6
<211> 24
<212> PRT
<213> Homo sapiens

<400> 6

Asp Val Leu Ala Gly Leu Ser Ser Ser Cys Cys Lys Trp Gly Cys Ser
1               5                   10                  15

Lys Ser Glu Ile Ser Ser Leu Cys
            20

<210>  7
<211>  24
<212>  PRT
<213>  Homo sapiens

<400>  7

Arg Pro Tyr Val Ala Leu Phe Glu Lys Cys Cys Leu Ile Gly Cys Thr
1               5                   10                  15

Lys Arg Ser Leu Ala Lys Tyr Cys
            20

<210>  8
<211>  24
<212>  PRT
<213>  Homo sapiens

<400>  8

Gln Leu Tyr Ser Ala Leu Ala Asn Lys Cys Cys His Val Gly Cys Thr
1               5                   10                  15

Lys Arg Ser Leu Ala Arg Phe Cys
            20

<210>  9
<211>  22
<212>  PRT
<213>  Homo sapiens

<400>  9

Ala Thr Asn Pro Ala Arg Tyr Cys Cys Leu Ser Gly Cys Thr Gln Gln
1               5                   10                  15

Asp Leu Leu Thr Leu Cys
            20

<210>  10
<211>  20
<212>  PRT
<213>  Homo sapiens

<400>  10

Gly Tyr Ser Glu Lys Cys Cys Leu Thr Gly Cys Thr Lys Glu Glu Leu
1               5                   10                  15

Ser Ile Ala Cys
            20

<210>  11
<211>  38
<212>  DNA
<213>  Artificial

<220>
<223>  sense primer

<400>  11
gatatcgccg ccaccatgca gatggccgat gcagccac                              38

<210>  12
<211>  28
<212>  DNA
<213>  Artificial

<220>
<223>  antisense primer

<400>  12
gatatctcag taggcagagc tgctgggc                                         28

<210>  13
<211>  5020
<212>  DNA
<213>  Artificial

<220>
<223>  pBabe Puro

<400>  13
ctgcagcctg aatatgggcc aaacaggata tctgtggtaa gcagttcctg ccccggctca      60

gggccaagaa cagatggaac agctgaatat gggccaaaca ggatatctgt ggtaagcagt     120

tcctgccccg gctcagggcc aagaacagat ggtccccaga tgcggtccag ccctcagcag     180

tttctagaga accatcagat gtttccaggg tgccccaagg acctgaaatg accctgtgcc     240

ttatttgaac taaccaatca gttcgcttct cgcttctgtt cgcgcgcttc tgctccccga     300

gctcaataaa agagcccaca acccctcact cggggcgcca gtcctccgat tgactgagtc     360

gcccgggtac ccgtgtatcc aataaaccct cttgcagttg catccgactt gtggtctcgc     420

tgttccttgg gagggtctcc tctgagtgat tgactacccg tcagcggggg tctttcattt     480

gggggctcgt ccgggatcgg gagacccctg cccagggacc accgacccac caccgggagg     540

taagctggcc agcaacttat ctgtgtctgt ccgattgtct agtgtctatg actgatttta     600

tgcgcctgcg tcggtactag ttagctaact agctctgtat ctggcggacc cgtggtggaa     660

ctgacgagtt ctgaacaccc ggccgcaacc ctgggagacg tcccagggac tttgggggcc     720

gtttttgtgg cccgacctga ggaagggagt cgatgtggaa tccgaccccg tcaggatatg     780

tggttctggt aggagacgag aacctaaaac agttcccgcc tccgtctgaa tttttgcttt     840

cggtttggaa ccgaagccgc gcgtcttgtc tgctgcagca tcgttctgtg ttgtctctgt     900

ctgactgtgt ttctgtattt gtctgaaaat tagggccaga ctgttaccac tcccttaagt     960

ttgaccttag atcactggaa agatgtcgag cggctcgctc acaaccagtc ggtagatgtc    1020

aagaagagac gttgggttac cttctgctct gcagaatggc caacctttaa cgtcggatgg    1080

ccgcgagacg gcacctttaa ccgagacctc atcacccagg ttaagatcaa ggtcttttca    1140

cctggcccgc atggacaccc agaccaggtc ccctacatcg tgacctggga agccttggct    1200

tttgaccccc ctccctgggt caagcccttt gtacacccta agcctccgcc tcctcttctt    1260

ccatccgcgc cgtctctccc ccttgaacct cctctttcga ccccgcctca atcctccctt    1320

tatccagccc tcactccttc tctaggcgcc ggccggatcc cagtgtggtg gtacgtagga 1380

attcgccagc acagtggtcg acctgtggaa tgtgtgtcag ttagggtgtg gaaagtcccc 1440

aggctcccca gcaggcagaa gtatgcaaag catgcatctc aattagtcag caaccaggtg 1500

tggaaagtcc ccaggctccc cagcaggcag aagtatgcaa agcatgcatc tcaattagtc 1560

agcaaccata gtcccgcccc taactccgcc catcccgccc ctaactccgc ccagttccgc 1620

ccattctccg ccccatggct gactaatttt ttttatttat gcagaggccg aggccgcctc 1680

ggcctctgag ctattccaga agtagtgagg aggctttttt ggaggcctag gcttttgcaa 1740

acgctgcttg aggctgaagg tgcgttgctg gcgtttttcc ataggctccg cccccctgac 1800

gagcatcaca aaaatcgacg ctcaagtcag aggtggcgaa acccgacagg actataaaga 1860

taccaggcgt ttccccctgg aagctccctc gtgcgctctc ctgttccgac cctgccgctt 1920

accggatacc tgtccgcctt tctcccttcg ggaagcgtgg cgctttctca tagctcacgc 1980

tgtaggtatc tcagttcggt gtaggtcgtt cgctccaagc tgggctgtgt gcacgaaccc 2040

cccgttcagc ccgaccgctg cgccttatcc ggtaactatc gtcttgagtc caacccggta 2100

agacacgact tatcgccact ggcagcagcc actggtaaca ggattagcag agcgaggtat 2160

gtaggcggtg ctacagagtt cttgaagtgg tggcctaact acggctacac tagaaggaca 2220

gtatttggta tctgcgctct gctgaagcca gttaccttcg gaaaaagagt tggtagctct 2280

tgatccggca aacaaaccac cgctggtagc ggtggttttt ttgtttgcaa gcagcagatt 2340

acgatcgata aaataaaaga ttttatttag tctccagaaa aaggggggaa tgaaagaccc 2400

cacctgtagg tttggcaagc tagcttaagt aacgccattt tgcaaggcat ggaaaaatac 2460

ataactgaga atagagaagt tcagatcaag gtcaggaaca gatggaacag ctgaatatgg 2520

gccaaacagg atatctgtgg taagcagttc ctgccccggc tcagggccaa gaacagatgg 2580

aacagctgaa tatgggccaa acaggatatc tgtggtaagc agttcctgcc ccggctcagg 2640

gccaagaaca gatggtcccc agatgcggtc cagccctcag cagtttctag agaaccatca 2700

gatgtttcca gggtgcccca aggacctgaa atgaccctgt gccttatttg aactaaccaa 2760

tcagttcgct tctcgcttct gttcgcgcgc ttctgctccc cgagctcaat aaaagagccc 2820

acaacccctc actcggggcg ccagtcctcc gattgactga gtcgcccggg tacccgtgta 2880

tccaataaac cctcttgcag ttgcatccga cttgtggtct cgctgttcct tgggagggtc 2940

tcctctgagt gattgactac ccgtcagcgg gggtctttca catgcagcat gtatcaaaat 3000

taatttggtt ttttttctta agtatttaca ttaaatggcc atagttgcat taatgaatcg 3060

gccaacgcgc ggggagaggc ggtttgcgta ttgggcgctc ttccgcttcc tcgctcactg 3120

actcgctgcg ctcggtcgtt cggctgcggc gagcggtatc agctcactca aaggcggtaa 3180

tacggttatc cacagaatca ggggataacg caggaaagaa catgtgagca aaaggccagc 3240

aaaaggccag gaaccgtaaa aaggccgcgt tgctggcgtt tttccatagg ctccgccccc 3300

ctgacgagca tcacaaaaat cgacgctcaa gtcagaggtg gcgaaacccg acaggactat 3360

aaagatacca ggcgtttccc cctggaagct ccctcgtgcg ctctcctgtt ccgaccctgc 3420

cgcttaccgg atacctgtcc gcctttctcc cttcgggaag cgtggcgctt tctcatagct 3480

cacgctgtag gtatctcagt tcggtgtagg tcgttcgctc caagctgggc tgtgtgcacg 3540

aaccccccgt tcagcccgac cgctgcgcct tatccggtaa ctatcgtctt gagtccaacc 3600

cggtaagaca cgacttatcg ccactggcag cagccactgg taacaggatt agcagagcga 3660

ggtatgtagg cggtgctaca gagttcttga agtggtggcc taactacggc tacactagaa 3720

ggacagtatt tggtatctgc gctctgctga agccagttac cttcggaaaa agagttggta 3780

gctcttgatc cggcaaacaa accaccgctg gtagcggtgg tttttttgtt tgcaagcagc 3840

agattacgcg cagaaaaaaa ggatctcaag aagatccttt gatcttttct acggggtctg 3900

acgctcagtg gaacgaaaac tcacgttaag ggattttggt catgagatta tcaaaaagga 3960

tcttcaccta gatcctttta aattaaaaat gaagtttgcg gccgcaaatc aatctaaagt 4020

atatatgagt aaacttggtc tgacagttac caatgcttaa tcagtgaggc acctatctca 4080

gcgatctgtc tatttcgttc atccatagtt gcctgactcc ccgtcgtgta gataactacg 4140

atacgggagg gcttaccatc tggccccagt gctgcaatga taccgcgaga cccacgctca 4200

ccggctccag atttatcagc aataaaccag ccagccggaa gggccgagcg cagaagtggt 4260

cctgcaactt tatccgcctc catccagtct attaattgtt gccgggaagc tagagtaagt 4320

agttcgccag ttaatagttt gcgcaacgtt gttgccattg ctacaggcat cgtggtgtca 4380

cgctcgtcgt ttggtatggc ttcattcagc tccggttccc aacgatcaag gcgagttaca 4440

tgatccccca tgttgtgcaa aaaagcggtt agctccttcg gtcctccgat cgttgtcaga 4500

agtaagttgg ccgcagtgtt atcactcatg gttatggcag cactgcataa ttctcttact 4560

gtcatgccat ccgtaagatg cttttctgtg actggtgagt actcaaccaa gtcattctga 4620

gaatagtgta tgcggcgacc gagttgctct tgcccggcgt caacacggga taataccgcg 4680

ccacatagca gaactttaaa agtgctcatc attggaaaac gttcttcggg gcgaaaactc 4740

tcaaggatct taccgctgtt gagatccagt tcgatgtaac ccactcgtgc acccaactga 4800

tcttcagcat cttttacttt caccagcgtt tctgggtgag caaaaacagg aaggcaaaat 4860

gccgcaaaaa agggaataag ggcgacacgg aaatgttgaa tactcatact cttccttttt 4920

caatattatt gaagcattta tcagggttat tgtctcatga gcggatacat atttgaatgt 4980

atttagaaaa ataaacaaat aggggttccg cgcacatttc 5020

<210> 14
<211> 83
<212> DNA
<213> Artificial

<220>
<223> sense strand for CREx2hb

<400> 14
cccaagcttg atatcgaatt cgacgtcaca gtatgacggc catgggaatt cgacgtcaca 60

gtatgacggc catggggatc ccg 83

<210> 15
<211> 83
<212> DNA
<213> Artificial

<220>
<223>  antisense strand for CREx2hb

<400>  15
cgggatcccc atggccgtca tactgtgacg tcgaattccc atggccgtca tactgtgacg      60

tcgaattcga tatcaagctt ggg                                              83


<210>  16
<211>  78
<212>  DNA
<213>  Artificial

<220>
<223>  sense strand for CREx2bp

<400>  16
tgcactgcag gaattcccat ggccgtcata ctgtgacgtc gaattcccat ggccgtcata      60

ctgtgacgtc ggatcccg                                                    78


<210>  17
<211>  78
<212>  DNA
<213>  Artificial

<220>
<223>  antisense strand for CREx2bp

<400>  17
cgggatccga cgtcacagta tgacggccat gggaattcga cgtcacagta tgacggccat      60

gggaattcct gcagtgca                                                    78


<210>  18
<211>  30
<212>  DNA
<213>  Artificial

<220>
<223>  sense primer

<400>  18
tcgactgcag cccatggccg tcatactgtg                                       30

<210> 19
<211> 30
<212> DNA
<213> Artificial

<220>
<223> antisense primer

<400> 19
tgcactgcag gtcggagctg actgttctgg 30

<210> 20
<211> 264
<212> DNA
<213> Artificial

<220>
<223> Vasoactive intestinal peptide promoter

<400> 20
tcgactgcag cccatggccg tcatactgtg tgacgtcttt cagagcactt tgtgattgct 60

cagtcctaag tataagccct ataaaatgat gggctttgaa atgctggtca gggtagagtg 120

agaagcacca gcaggcagta acagccaacc cttagccatt gctaagggca gagaactggt 180

ggagcctttc tcttactccc aggacttcag cacctaagac agctccaaaa caaaccagaa 240

cagtcagctc cgacctgcag tgca 264

## Description

### Technical Field

The present invention relates to a polypeptide having an antianxiety activity; a method of treating anxiety using the polypeptide; a method of screening for a compound, a salt thereof, or a hydrate of them which is involved in the regulation of anxiety and activates or suppresses a receptor of the polypeptide; and a kit for the screening.

### Background Art

Physiologically active substances, such as brain-gut hormones, chemokines, neuropeptides, and neurotransmitters, exhibit their functions via specific receptors present in the cell membrane. Of these receptors, receptors which have a structure to penetrate the cell membrane seven times and are coupled with the G protein trimer in the cells are particularly classified as G-protein-coupled receptors (GPCRs). Upon binding with specific ligands, the GPCRs transmit signals into the cells to activate or suppress the cells and thus play an important role in expressing functions in various organs. Therefore, agonists which activate GPCRs and antagonists which suppress GPCRs have been used as medicines. Of receptors classified into GPCRs, many for which no specific ligand has been identified are known and called orphan GPCRs. The orphan GPCRs have a potential to become a target for novel therapeutic agents, and thus identification of their ligands and research on substances to activate or suppress their function have been in progress. It is extremely important in developing new medicines to elucidate functions of the receptors and their ligands by administering the identified ligands or substances to the body.

In recent years, enrichment of the genetic sequence information makes it possible to predict and identify an unknown peptide or protein as a novel GPCR ligand by deducing its homology and regularity based on sequences of known proteins or peptides. Relaxin, a member of the insulin/relaxin family, is a secretory hormone produced by the corpus luteum or the placenta and has long been known to have functions involved in the maintenance of pregnancy and the delivery. A protein encoded by a DNA sequence which is newly identified by a gene sequence database based on the base sequence of DNA encoding relaxin is a polypeptide called relaxin-3/INSL7 (WO 01/068862). A mature- or activate-form of relaxin-3 is composed of a B-chain and a A-chain which are excised from a preproprotein of relaxin-3 and the B-chain and the A-chain are bonded through disulfide bonds. Relaxin-3 thus found has been reported to activate cells with an increase in intracellular cyclic AMP (cAMP) of THP-1 cells of the immune system (WO 01/81562, Bathgate et al., J. Biol. Chem., 277, p.1148-1157, 2002). It has later been suggested that relaxin-3, along with relaxin-2, is one of ligands which bind LGR7, a GPCR, and that LGR7 is involved in the increase of cAMP by relaxin-3 (Sudo et al., J. Biol. Chem., 278, p.7855-7862, 2003). LGR7 is expressed in the brain and peripheral tissues and has been so far suggested to be involved in development of reproductive organs, pregnancy, and delivery; however, its correlation with neurologic manifestation has not clearly been understood.

Recently it has been reported that a ligand for GPCRs for which no ligand in the body has been identified, i.e., a receptor called SALPR (GPCR135) and a receptor called GPR100 (hGPCR11, GPCR142), is relaxin-3 (Takeda et al., FEBS Letter, 520, p.97-101, 2002, Liu et al., J. Biol. Chem., 278, p.50754-50764, 2003; Liu et al., J. Biol. Chem., 278, p.50765-50770, 2003; and WO 2004/082598). It has been reported that SALPR (Liu et al., J. Biol. Chem., 278, p.50754-50764, 2003) and GPR100 (Liu et al., J. Biol, Chem., 278, p.50765-50770, 2003) are involved in decrease in cAMP by relaxin-3. Further, WO 00/24891, WO 01/48189, WO 02/31111, and WO 02/610877 also include descriptions related to these receptors. SALPR is known to locate in the brain (Matsumoto et al., Gene, 248, p.183-189, 2000), and in particular reported to locate in the paraventricular nucleus and the supraoptic nucleus of the hypothalamus (WO 2004/082598, Liu et al., J. Biol. Chem., 278, p.50754-50764, 2003). In addition, the expression of SALPR has been studied by identifying the binding region of a peptide in the brain using a chimeric peptide between relaxin-3 and INSL5 which selectively binds to SALPR (Sutton et al., Neuroendocrinology, 180, p.298-307, 2004); however, its function still remains unknown. GPR100 has been reported to be a receptor which is systemically expressed (Liu et al., J. Biol. Chem., 278, p.50765-50770, 2003, Boels et al., Br J. Pharamacol., 140, p.932-938, 2003); however, its function also still remains unknown,

On the other hand, relaxin-3 has been reported to be present in a specific area in the brain (Liu et al., J. Biol. Chem., 278, p.50754-50764, 2003) and it has been thought that relaxin-3 may exhibit some functions as an intracerebral peptide in the central nervous system; however, there has been no report on whether relaxin-3 regulates mental conditions such as anxiety and depression.

### Summary of the Invention

Accordingly, an object of the present invention is to provide a polypeptide having an antianxiety activity; a therapeutic agent containing the polypeptide; a method of treating anxiety using the polypeptide; a method of screening for a compound, a salt thereof, or a hydrate of them which is involved in the regulation of anxiety and activates or suppresses a receptor of the polypeptide; and a kit for the screening.

The present inventors found that relaxin-3 has an antianxiety activity as a result of intracerebroventricularly administering relaxin-3 to rats or mice in an experimental system for evaluating anxiogenic activities and antianxiety activities, and observing the behaviors of the rats or mice after the administration. The present invention has been made based on these findings.

Specifically, according to the present invention, there is provided:
(1) an antianxiety agent containing relaxin-3, a salt thereof, or a hydrate of them;
(2) the antianxiety agent according to (1), in which relaxin-3 is human relaxin-3;
(3) the antianxiety agent according to (1), wherein relaxin-3 is a polypeptide consisting of an A-chain and a B-chain which are obtainable from a functionally equivalent modified polypeptide of a relaxin-3 preproprotein, or consisting of an A-chain and a B-chain which are obtainable from a homologous polypeptide of a relaxin-3 preproprotein, and wherein cysteine residues of the A-chain and the B-chain are bonded through disulfide bonds;
(4) a method of screening for a compound having an antianxiety activity, a salt thereof, or a hydrate of them, the method including the steps of:
   (A) contacting a test substance with a relaxin-3 receptor, a cell containing a relaxin-3 receptor, or a membrane fraction of the cell; and
   (B) measuring a cell-stimulating activity via the relaxin-3 receptor;
(4') a method of screening for a compound having an antianxiety activity, a salt thereof, or a hydrate of them, the method including the steps of:
   (A) contacting a test substance with a relaxin-3 receptor, a cell containing a relaxin-3 receptor, or a membrane fraction of the cell,
   (B) measuring a cell-stimulating activity via the relaxin-3 receptor, and
   (C) determining that the test substance is a compound having an activity of suppressing an anxiety activity when the cell-stimulating activity via the relaxin-3 receptor, such as somatostatin- and angiogenin-like peptide receptor (SALPR), shows suppression of adenylate cyclase activity;
(5) a method of screening for a compound suppressing or stimulating an anxiety activity, a salt thereof, or a hydrate of them, the method including the step of:
   (A) contacting a test substance and relaxin-3, a salt thereof, or a hydrate of them with a relaxin-3 receptor, a cell containing a relaxin-3 receptor, or a membrane fraction of the cell;
(6) the screening method according to (5), in which relaxin-3 is human relaxin-3;
(7) the screening method according to (5), wherein relaxin-3 is a polypeptide consisting of an A-chain and a B-chain which are obtainable from a functionally equivalent modified polypeptide of a relaxin-3 preproprotein, or consisting of an A-chain and a B-chain which are obtainable from a homologous polypeptide of a relaxin-3 preproprotein, and wherein cysteine residues of the A-chain and the B-chain are bonded through disulfide bonds;
(8) the method of screening for a compound suppressing or stimulating an anxiety activity, a salt thereof, or a hydrate of them according to any one of (5) to (7), further including the step of:
   (B) measuring a cell-stimulating activity via the relaxin-3 receptor;
(8') the method of screening for a compound suppressing anxiety, a salt thereof, or a hydrate of them according to any one of (5) to (7), further including the steps of:
   (B) measuring a cell-stimulating activity via the relaxin-3 receptor, and
   (C) determining that the test substance is a compound having an activity of suppressing an anxiety activity when the cell-stimulating activity via the relaxin-3 receptor, such as SALPR, shows the suppression of an adenylate cyclase activity;
(9) the screening method according to any one of (4), (4'), (5), (6), (7), (8), and (8'), in which the relaxin-3 receptor is SALPR or a partial polypeptide thereof;
(10) the screening method according to (9), in which the SALPR is a polypeptide containing the amino acid sequence represented by SEQ ID NO: 4;
(11) a kit for screening for a compound having an antianxiety activity, a salt thereof, or a hydrate of them, the kit including a relaxin-3 receptor, a cell containing a relaxin-3 receptor, or a membrane fraction of the cell;
(12) the screening kit according to (11), which further contains relaxin-3, a salt thereof, or a hydrate of them;
(13) the screening kit according to (12), in which relaxin-3 is human relaxin-3;
(14) the screening kit according to (12), wherein relaxin-3 is a polypeptide consisting of an A-chain and a B-chain which are obtainable from a functionally equivalent modified polypeptide of a relaxin-3 preproprotein, or consisting of an A-chain and a B-chain which are obtainable from a homologous polypeptide of a relaxin-3 preproprotein, and wherein cysteine residues of the A-chain and the B-chain are bonded through disulfide bonds;
(15) the screening kit according to any one of (12) to (14), in which relaxin-3 is labeled;
(16) the screening kit according to any one of (11) to (15), in which the relaxin-3 receptor is SALPR or a partial polypeptide thereof;
(17) the screening kit according to (16), in which the SALPR is a polypeptide including the amino acid sequence represented by SEQ ID NO: 4;
(18) a method of screening for a compound suppressing or stimulating an anxiety activity, a salt thereof, or a hydrate of them, the method including the steps of administering a compound acting on a relaxin-3 receptor to a human or a non-human organism, and measuring an anxiety activity after administration;
(19) the screening method according to (18), in which the step of measuring an anxiety activity includes carrying out a defensive burying test or an elevated plus-maze test; and
(20) the screening method according to one of (18) and (19), in which the compound acting on a relaxin-3 receptor is a compound obtained through the method of any one of (4), (4'), (5), (6), (7), (8), (8'), (9), and (10).

### Brief Description of the Drawings

Fig. 1 illustrates the construction of pBabeCL (SALPR) IH.
Fig. 2A illustrates the construction of CRE4VTP/pBluescriptIISK(+) .
Fig. 2B illustrates the construction of pBabeCLX.
Fig. 2C illustrates the construction of pBabeCLcre4vPdNN.

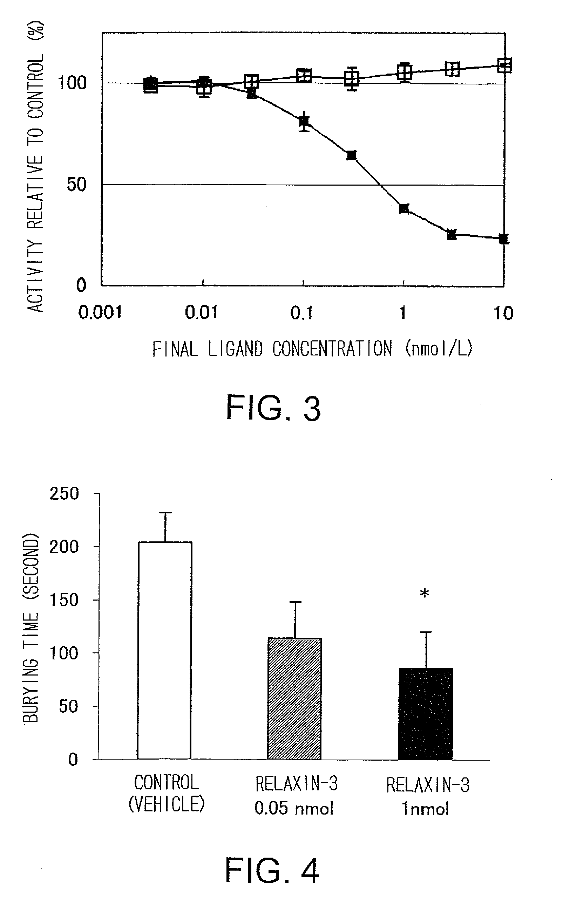

Fig. 3 shows specific dose-dependent suppression by relaxin-3 of transcription activity which is increased by the addition of forskolin in SE302 cells in which SALPR is expressed. Filled squares show data where relaxin-3 was added. Open squares show data where insulin was added. The numbers on the abscissa show the final concentration (nmol/L) of each ligand added. The numbers on the ordinate show the relative activity calculated by setting alkaline phosphatase activity of cellular supernatant with the addition of forskolin at 1 µmol/L to be 100 and with no forskolin to be 0. Each point shows the mean (N=3) and standard deviation.
Fig. 4 shows the antianxiety activity of a single intracerebroventricular administration of relaxin-3 to rats as determined by a defensive burying test. The open bar shows a control (vehicle) administration group, the diagonally shaded bar shows a 0.05-nmol relaxin-3 administration group, and the filled bar shows a 1-nmol relaxin-3 administration group. The ordinate indicates the mean and standard error of the time (second) within which a test animal shows a behavior of burying an electrode with a bedding material, per animal in each group. The asterisk (*) in Fig. 4 means that the human relaxin-3 administration group shows a significant difference versus the control (vehicle) administration group (Dunnett multiple comparison test, P<0.05).
Fig. 5 shows the total number of entries into open and closed arms in an elevated plus-maze test (5 minutes) using mice.
Fig. 6 shows the ratio of the time spent in open arms in an elevated plus-maze test (5 minutes) using mice. The asterisk (*) in Fig. 6 means that the human relaxin-3 administration group shows a significant difference versus the control (vehicle) administration group (t-test, P<0.05).
Fig. 7 shows the number of entries into open and closed arms in an elevated plus-maze test using rats.
Fig. 8 shows the ratio of the time spent in open arms in an elevated plus-maze test (5 minutes) using rats. The asterisk (*) in Fig. 8 means that the human relaxin-3 administration group shows a significant difference versus the control (vehicle) administration group (Dunnett multiple comparison test, P<0.05).

### Detailed Description of the Invention

### Relaxin-3

"Relaxin-3" used in the present invention is a polypeptide called relaxin-3 (also known as INSL7) and means a mature- or active-form relaxin-3.

Specifically, the term "relaxin-3" used in the present invention means a polypeptide having a polypeptide of the amino acid sequence of the 26th (Arg) to the 52nd (Trp) residues from the N-terminus of SEQ ID NO: 2; a modified polypeptide which is functionally equivalent to the polypeptide; or a polypeptide which is homologous to the polypeptide (hereinafter also simply abbreviated as "B-chain'') and a polypeptide of the amino acid sequence of the 119th (Asp) to the 142nd (Cys) residues from the N-terminus of SEQ ID NO: 2; a modified polypeptide which is functionally equivalent to the polypeptide; or a polypeptide which is homologous to the polypeptide (hereinafter also simply abbreviated as "A-chain"), in which cysteine residues of the B-chain and the A-chain are bonded through disulfide bonds. The cysteine residues of the B-chain and the A-chain are preferably intermolecularly and intramolecularly bonded through disulfide bonds.

More specifically, relaxin-3 used in the present invention means a polypeptide containing a polypeptide of the amino acid sequence of the 26th (Arg) to the 52nd (Trp) residues from the N-terminus of SEQ ID NO: 2 (human B-chain) and a polypeptide of the amino acid sequence of the 119th (Asp) to the 142nd (Cys) residues from the N-terminus of SEQ ID NO: 2 (human A-chain), wherein the two polypeptides are bonded through disulfide bonds, and wherein cysteine residues of the B-chain and the A-chain intermolecularly and intramolecularly form disulfide bonds. The disulfide bonds are desirably that cysteine in B-chain at the 35th position from the N-terminus of SEQ ID NO: 2 is bonded to cysteine in A-chain at the 129th position from the N-terminus of SEQ ID NO: 2; cysteine in B-chain at the 47th position from the N-terminus of SEQ ID NO: 2 is bonded to cysteine in A-chain at the 142nd position from the N-terminus of SEQ ID NO: 2; and cysteine in A-chain at the 128th position from the N-terminus of SEQ ID NO: 2 is bonded to cysteine in A-chain at the 133rd position from the N-terminus of SEQ ID NO: 2.

Examples of relaxin-3 used in the present invention are as follows. The numerals herein represent cysteine residue numbers involved in disulfide bonds, and the cysteine residues with the same residue number are bonded to each other through disulfide bond.

### [Human Relaxin-3]

B-chain: RAAPYGVRLCGREFIRAVIFTCGGSRW (SEQ ID NO: 5)
1 2

A-chain: DVLAGLSSSCCKWGCSKSEISSLC (SEQ ID NO: 6)
31 3 2

The amino acid sequences of the B-chain and the A-chain are contained in the amino acid sequence of a preproprotein of relaxin-3 used in the present invention. The preproprotein of relaxin-3 used in the present invention can be a polypeptide of the amino acid sequence represented by SEQ ID NO: 2 (human preproprotein) (GenBank Accession Number NM-080864), a functionally equivalent modified polypeptide of the polypeptide, or a homologous polypeptide of the polypeptide (these are hereinafter also simply abbreviated as "preproprotein"). Relaxin-3 used in the present invention further includes a polypeptide containing a B-chain and an A-chain cleaved from the preproprotein, in which cysteine residues in the B-chain and A-chain are bonded through disulfide bonds.

Such relaxin-3, B-chain, A-chain, and preproprotein used in the present invention can be any of naturally occurring polypeptides derived from, for example, humans and non-human organisms including non-human mammals (e.g., mice, rats, hamsters, pigs, and canines), birds, reptiles, amphibians, fish, and insects; recombinant polypeptides; and synthetic polypeptide. Relaxin-3 used in the present invention further includes salts of relaxin-3, including those with or without sugar chains. The salts will be described in later. Relaxin-3, B-chain, A-chain, and the preproprotein used in the present invention further include polypeptides that have undergone secretory protein processing, such as N-terminal cyclic glutamination and C-terminal amidation.

The term "functionally equivalent modified polypeptide" as used herein means a polypeptide which has a polypeptide of the amino acid sequence of the 26th (Arg) to the 52nd (Trp) residues from the N-terminus of SEQ ID NO: 2 (human B-chain), a polypeptide of the amino acid sequence of the 119th (Asp) to the 142nd (Cys) residues from the N-terminus of SEQ ID NO: 2 (human A-chain), or a polypeptide of the amino acid sequence represented by SEQ ID NO: 2 (human preproprotein), wherein one or more (preferably one or several) amino acids are deleted, substituted, inserted and/or added, wherein cysteine residues in B-chain and A-chain are bonded through disulfide bonds, and wherein it exhibits substantially the same activities as relaxin-3 [for example relaxin-3-receptor binding ability, various cell-stimulating activities associated with the binding (e.g., intracellular calcium Ca²⁺ release, adenylyl cyclase activation, intracellular cAMP production, intracellular cGMP production, inositol phospholipid production, electrical potential change in the cell membrane, pH change in the vicinity of the cell membrane, phosphorylation of intracellular proteins, c-fos and c-jun induction/activation, and arachidonic acid release), and regulation of an anxiety activity]. The functionally equivalent modified polypeptide can be any of the above-mentioned organism-derived polypeptides, recombinant polypeptides, and synthetic polypeptides, as long as it satisfies the above conditions.

The deletion, substitution and/or insertion can occur at any position in the amino acid sequence, but may occur at amino acid residues other than cysteine residues in the amino acid sequence of a polypeptide having the amino acid sequence of the 26th (Arg) to the 52nd (Trp) residues from the N-terminus of SEQ ID NO: 2 (human B-chain), a polypeptide having the amino acid sequence of the 119th (Asp) to the 142nd (Cys) residues from the N-terminus of SEQ ID NO: 2 (human A-chain), or a polypeptide having the amino acid sequence represented by SEQ ID NO: 2 (human preproprotein).

The term "substitution" in this specification preferably means a conservative substitution of one or more amino acid residues with other chemically homologous amino acid residues, so as not to substantially change peptide activity. For example, a certain hydrophobic residue can be substituted with another hydrophobic residue, and a certain polar residue can be substituted with another polar residue having the same charge. Functionally homologous amino acids capable of carrying out these substitutions for each amino acid are known to those skilled in the art. More specifically, examples of non-polar (hydrophobic) amino acids include alanine, valine, isoleucine, leucine, proline, tryptophan, phenylalanine, and methionine. Examples of polar (neutral) amino acids include glycine, serine, threonine, tyrosine, glutamine, asparagine, and cysteine. Examples of positively charged (basic) amino acids include arginine, histidine, and lysine. Examples of negatively charged (acidic) amino acids include aspartic acid and glutamic acid.

The number of amino acid residues to be deleted, substituted, inserted, and/or added is, for example, 1 to 30, preferably 1 to 20, more preferably 1 to 10, further more preferably 1 to 5, and most preferably 1 or 2.

The term "homologous polypeptide" refers to a polypeptide which has an amino acid sequence having 70% or more, preferably 80% or more, more preferably 85% or more, further preferably 90% or more, further more preferably 95% or more, particularly preferably 98% or more, and most preferably 99% or more, homology to the amino acid sequence of a polypeptide having the amino acid sequence of the 26th (Arg) to the 52nd (Trp) residues from the N-terminus of SEQ ID NO: 2 (human B-chain), a polypeptide of the amino acid sequence of the 119th (Asp) to the 142nd (Cys) residues from the N-terminus of SEQ ID NO: 2 (human A-chain), or a polypeptide having the amino acid sequence represented by SEQ ID NO: 2 (human preproprotein), wherein cysteine residues in the B-chain and the A-chain are bonded through disulfide bonds, and wherein it exhibits substantially the same activities as relaxin-3 used in the present invention (for example, relaxin-3-receptor binding ability, various cell-stimulating activities associated with the binding, and regulation of an antianxiety activity). The homologous polypeptide is not particularly limited, but can be any of the organism-derived polypeptides, recombinant polypeptides, and synthetic polypeptides, as long as it exhibits the above activities.

The figures for the "homology" (also referred to as "identity") in this specification can be figures calculated using a homology search program known to those skilled in the art; for example, they can be calculated using default parameters in the homology algorithm BLAST (basic local alignment search tool) http://www.ncbi.nlm.nih.gov/BLAST/ by The National Center for Biotechnology Information (NCBI).

Preferred examples of B-chain, A-chain, relaxin-3, and the preproprotein in the functionally equivalent modified polypeptide and homologous polypeptide include known murine-, rat-, or swine-originated B-chains, A-chains, relaxin-3, and preproproteins (WO 01/81562); and preproproteins and B-chains of relaxin-1, relaxin-2, and insulin-like peptide 3 (INSL-3) (WO 2006/026355).

The "polypeptide consisting of an A-chain and a B-chain which are obtainable from a functionally equivalent modified polypeptide of a relaxin-3 preproprotein, or consisting of an A-chain and a B-chain which are obtainable from a homologous polypeptide of a relaxin-3 preproprotein, wherein cysteine residues of the A-chain and the B-chain are bonded through disulfide bonds" is preferably one of the following polypeptides (1) and (2) which exhibit substantially the same activities as relaxin-3 used in the present invention (for example, relaxin-3-receptor binding ability, various cell-stimulating activities associated with the binding, and regulation of an antianxiety activity):
(1) a polypeptide which consists of a polypeptide of the amino acid sequence represented by SEQ ID NO: 5 (human B-chain) or a modified human B-chain in which one or more (preferably one or several, more preferably one, two, three, or four, further preferably one or two, and particularly preferably one) amino acids have been deleted, substituted, inserted, and/or added in the amino acid sequence of SEQ ID NO: 5 and a polypeptide of the amino acid sequence represented by SEQ ID NO: 6 (human A-chain) or a modified human A-chain in which one or more (preferably one or several, more preferably one, two, three, or four, further preferably one or two, and particularly preferably one) amino acids have been deleted, substituted, inserted, and/or added in the amino acid sequence of SEQ ID NO: 6, wherein cysteine in B--chain at the 10th position from the N-terminus of SEQ ID NO: 5 is bonded to cysteine in A-chain at the 11th position from the N-terminus of SEQ ID NO: 6; cysteine in B-chain at the 22nd position from the N-terminus of SEQ ID NO: 5 is bonded to cysteine in A-chain at the 24th position from the N-terminus of SEQ ID NO: 6; and cysteine in A-chain at the 10th position from the N-terminus of SEQ ID NO: 6 is bonded to cysteine in A-chain at the 15th position from the N-terminus of SEQ ID NO: 6; and
(2) a polypeptide which consists of a polypeptide of the amino acid sequence represented by SEQ ID NO: 5 (human B-chain) or a homologous human B-chain which has an amino acid sequence with 70% or more (preferably 80% or more, more preferably 85% or more, further preferably 90% or more, further more preferably 95% or more, particularly preferably 98% or more, and most preferably 99% or more) homology to the amino acid sequence of human B-chain, and a polypeptide of the amino acid sequence represented by SEQ ID NO: 6 (human A-chain) or a homologous human A-chain which has an amino acid sequence with 70% or more (preferably 80% or more, more preferably 85% or more, further preferably 90% or more, further more preferably 95% or more, particularly preferably 98% or more, and most preferably 99% or more) homology to the amino acid sequence of human A-chain, wherein cysteine in B-chain at the 10th position from the N-terminus of SEQ ID NO: 5 is bonded to cysteine in A-chain at the 11th position from the N-terminus of SEQ ID NO: 6; cysteine in B-chain at the 22nd position from the N-terminus of SEQ ID NO: 5 is bonded to cysteine in A-chain at the 24th position from the N-terminus of SEQ ID NO: 6; and cysteine in A-chain at the 10th position from the N-terminus of SEQ ID NO: 6 is bonded to cysteine in A-chain at the 15th position from the N-terminus of SEQ ID NO: 6.
   Examples of the "polypeptide consisting of an A-chain and a B-chain which are obtainable from a functionally equivalent modified polypeptide of a relaxin-3 preproprotein, or consisting of an A-chain and a B-chain which are obtainable from a homologous polypeptide of a relaxin-3 preproprotein, wherein cysteine residues of the A-chain and the B-chain are bonded through disulfide bonds" include chimeric peptides of relaxin-3 disclosed in WO 2006/026355 and Changlu Liu et al., Mol Pharmacol. 67(1):231-40(2005).
   Preferred examples of such chimeric peptides of relaxin-3 include the following polypeptides (3) to (10) which have substantially the same activities as those of relaxin-3 used in the present invention (for example, relaxin-3-receptor binding ability, various cell-stimulating activities associated with the binding, and regulation of an antianxiety activity):
(3) a polypeptide which consists of a polypeptide of the amino acid sequence represented by SEQ ID NO: 5 (human B-chain) or a modified human B-chain in which one or more (preferably one or several, more preferably one, two, three, or four, further preferably one or two, and particularly preferably one) amino acids have been deleted, substituted, inserted, and/or added in the amino acid sequence of SEQ ID NO: 5, and a polypeptide of the amino acid sequence represented by SEQ ID NO: 7 (human relaxin-1 A-chain) or a modified human relaxin-1 A-chain in which one or more (preferably one or several, more preferably one, two, three, or four, further preferably one or two, and particularly preferably one) amino acids have been deleted, substituted, inserted, and/or added in the amino acid sequence of SEQ ID NO: 7, wherein cysteine in B-chain at the 10th position from the N-terminus of SEQ ID NO: 5 is bonded to cysteine in A-chain at the 11th position from the N-terminus of SEQ ID NO: 7; cysteine in B-chain at the 22nd position from the N-terminus of SEQ, ID NO: 5 is bonded to cysteine in A-chain at the 24th position from the N-terminus of SEQ ID NO: 7; and cysteine in A-chain at the 10th position from the N-terminus of SEQ ID NO: 7 is bonded to cysteine in A-chain at the 15th position from the N-terminus of SEQ ID NO: 7;
(4) a polypeptide which consists of a polypeptide of the amino acid sequence represented by SEQ ID NO: 5 (human B-chain) or a homologous human B-chain which has an amino acid sequence with 70% or more (preferably 80% or more, more preferably 85% or more, further preferably 90% or more, further more preferably 95% or more, particularly preferably 98% or more, and most preferably 99% or more) homology to the amino acid sequence of human B-chain, and a polypeptide of the amino acid sequence represented by SEQ ID NO: 7 (human relaxin-1, A-chain) or a homologous human relaxin-1 A-chain which has an amino acid sequence with 70% or more (preferably 80% or more, more preferably 85% or more, further preferably 90% or more, further more preferably 95% or more, particularly preferably 98% or more, and most preferably 99% or more) homology to the amino acid sequence of human relaxin-1 A-chain, wherein cysteine in B-chain at the 10th position from the N-terminus of SEQ ID NO: 5 is bonded to cysteine in A-chain at the 11th position from the N-terminus of SEQ ID NO: 7; cysteine in B-chain at the 22nd position from the N-terminus of SEQ ID NO: 5 is bonded to cysteine in A-chain at the 24th position from the N-terminus of SEQ ID NO: 7; and cysteine in A-chain at the 10th position from the N-terminus of SEQ ID NO: 7 is bonded to cysteine in A-chain at the 15th position from the N-terminus of SEQ ID NO: 7;
(5) a polypeptide which consists of a polypeptide of the amino acid sequence represented by SEQ ID NO: 5 (human B-chain) or a modified human B-chain in which one or more (preferably one or several, more preferably one, two, three, or four, further preferably one or two, and particularly preferably one) amino acids have been deleted, substituted, inserted, and/or added in the amino acid sequence of SEQ ID NO: 5, and a polypeptide of the amino acid sequence represented by SEQ ID NO: 8 (human relaxin-2 A-chain) or a modified human relaxin-2 A-chain in which one or more (preferably one or several, more preferably one, two, three, or four, further preferably one or two, and particularly preferably one) amino acids have been deleted, substituted, inserted, and/or added in the amino acid sequence of SEQ ID NO: 8, wherein cysteine in B-chain at the 10th position from the N-terminus of SEQ ID NO: 5 is bonded to cysteine in A-chain at the 11th position from the N-terminus of SEQ ID NO: 8; cysteine in B-chain at the 22nd position from the N-terminus of SEQ ID NO: 5 is bonded to cysteine in A-chain at the 24th position from the N-terminus of SEQ ID NO: 8; and cysteine in A-chain at the 10th position from the N-terminus of SEQ ID NO: 8 is bonded to cysteine in A-chain at the 15th position from the N-terminus of SEQ ID NO: 8;
(6) a polypeptide which consists of a polypeptide of the amino acid sequence represented by SEQ ID NO: 5 (human B-chain) or a homologous human B-chain which has an amino acid sequence with 70% or more (preferably 80% or more, more preferably 85% or more, further preferably 90% or more, further more preferably 95% or more, particularly preferably 98% or more, and most preferably 99% or more) homology to the amino acid sequence of human B-chain, and a polypeptide of the amino acid sequence represented by SEQ ID NO: 8 (human relaxin-2 A-chain) or a homologous human relaxin-2 A-chain which has an amino acid sequence with 70% or more (preferably 80% or more, more preferably 85% or more, further preferably 90% or more, further more preferably 95% or more, particularly preferably 98% or more, and most preferably 99% or more) homology to the amino acid sequence of human relaxin-2 A-chain, wherein cysteine in B-chain at the 10th position from the N-terminus of SEQ ID NO: 5 is bonded to cysteine in A-chain at the 11th position from the N-terminus of SEQ ID NO: 8; cysteine in B-chain at the 22nd position from the N-terminus of SEQ ID NO: 5 is bonded to cysteine in A-chain at the 24th position from the N-terminus of SEQ ID NO: 8; and cysteine in A-chain at the 10th position from the N-terminus of SEQ ID NO: 8 is bonded to cysteine in A-chain at the 15th position from the N-terminus of SEQ ID NO: 8;
(7) a polypeptide which consists of a polypeptide of the amino acid sequence represented by SEQ ID NO: 5 (human B-chain) or a modified human B-chain in which one or more (preferably one or several, more preferably one, two, three, or four, further preferably one or two, and particularly preferably one) amino acids have been deleted, substituted, inserted, and/or added in the amino acid sequence of SEQ ID NO: 5, and a polypeptide of the amino acid sequence represented by SEQ ID NO: 9 (modified A-chain of human insulin-like peptide 3) or an A-chain of modified human insulin-like peptide 3 in which one or more (preferably one or several, more preferably one, two, three, or four, further preferably one or two, and particularly preferably one) amino acids have been deleted, substituted, inserted, and/or added in the amino acid sequence of SEQ ID NO: 9, wherein cysteine in B-chain at the 10th position from the N-terminus of SEQ ID NO: 5 is bonded to cysteine in A-chain at the 9th position from the N-terminus of SEQ ID NO: 9; cysteine in B-chain at the 22nd position from the N-terminus of SEQ ID NO: 5 is bonded to cysteine in A-chain at the 22nd position from the N-terminus of SEQ ID NO: 9; and cysteine in A-chain at the 8th position from the N-terminus of SEQ ID NO: 9 is bonded to cysteine in A-chain at the 13th position from the N-terminus of SEQ ID NO: 9;
(8) a polypeptide which consists of a polypeptide of the amino acid sequence represented by SEQ ID NO: 5 (human B-chain) or a homologous human B-chain which has an amino acid sequence with 70% or more (preferably 80% or more, more preferably 85% or more, further preferably 90% or more, further more preferably 95% or more, particularly preferably 98% or more, and most preferably 99% or more) homology to the amino acid sequence of human B-chain, and a polypeptide of the amino acid sequence represented by SEQ ID NO: 9 (modified A-chain of human insulin-like peptide 3) or an A-chain of homologous human insulin-like peptide 3 which has an amino acid sequence with 70% or more (preferably 80% or more, more preferably 85% or more, further preferably 90% or more, further more preferably 95% or more, particularly preferably 98% or more, and most preferably 99% or more) homology to the amino acid sequence of the modified A-chain of human insulin-like peptide 3, wherein cysteine in B-chain at the 10th position from the N-terminus of SEQ ID NO: 5 is bonded to cysteine in A-chain at the 9th position from the N-terminus of SEQ ID NO: 9; cysteine in B-chain at the 22nd position from the N-terminus of SEQ ID NO: 5 is bonded to cysteine in A-chain at the 22nd position from the N-terminus of SEQ ID NO: 9; and cysteine in A-chain at the 8th position from the N-terminus of SEQ ID NO: 9 is bonded to cysteine in A-chain at the 13th position from the N-terminus of SEQ ID NO: 9;
(9) a polypeptide which consists of a polypeptide of the amino acid sequence represented by SEQ ID NO: 5 (human B-chain) or a modified human B-chain in which one or more (preferably one or several, more preferably one, two, three, or four, further preferably one or two, and particularly preferably one) amino acids have been deleted, substituted, inserted, and/or added in the amino acid sequence of SEQ ID NO: 5, and a polypeptide of the amino acid sequence represented by SEQ ID NO: 10 (modified A-chain of human insulin-like peptide 6) or an A-chain of modified human insulin-like peptide 6 in which one or more (preferably one or several, more preferably one, two, three, or four, further preferably one or two, and particularly preferably one) amino acids have been deleted, substituted, inserted, and/or added in the amino acid sequence of SEQ ID NO: 10, wherein cysteine in B-chain at the 10th position from the N-terminus of SEQ ID NO: 5 is bonded to cysteine in A-chain at the 7th position from the N-terminus of SEQ ID NO: 10; cysteine in B-chain at the 22nd position from the N-terminus of SEQ ID NO: 5 is bonded to cysteine in A-chain at the 20th position from the N-terminus of SEQ ID NO: 10; and cysteine in A-chain at the 6th position from the N-terminus of SEQ ID NO: 10 is bonded to cysteine in A-chain at the 11th position from the N-terminus of SEQ ID NO: 10; and
(10) a polypeptide which consists of a polypeptide of the amino acid sequence represented by SEQ ID NO: 5 (human B-chain) or a homologous human B-chain which has an amino acid sequence with 70% or more (preferably 80% or more, more preferably 85% or more, further preferably 90% or more, further more preferably 95% or more, particularly preferably 98% or more, and most preferably 99% or more) homology to the amino acid sequence of human B-chain, and a polypeptide of the amino acid sequence represented by SEQ ID NO: 10 (modified A-chain of human insulin-like peptide 6) or an A-chain of homologous human insulin-like peptide 6 which has an amino acid sequence with 70% or more (preferably 80% or more, more preferably 85% or more, further preferably 90% or more, further more preferably 95% or more, particularly preferably 98% or more, and most preferably 99% or more) homology to the amino acid sequence of the modified A-chain of human insulin-like peptide 6, wherein cysteine in B-chain at the 10th position from the N-terminus of SEQ ID NO: 5 is bonded to cysteine in A-chain at the 7th position from the N-terminus of SEQ ID NO: 10; cysteine in B-chain at the 22nd position from the N-terminus of SEQ ID NO: 5 is bonded to cysteine in A-chain at the 20th position from the N-terminus of SEQ ID NO: 10; and cysteine in A-chain at the 6th position from the N-terminus of SEQ ID NO: 10 is bonded to cysteine in A-chain at the 11th position from the N-terminus of SEQ ID NO: 10.

More preferred examples of chimeric peptides of relaxin-3 include the following polypeptides. The numerals herein represent cysteine residues bonded through disulfide bonds, and the cysteine residues with an identical numeral are bonded to each other through disulfide bond. These chimeric peptides have been verified to have ligand activities to SALPR (GPCR135), GPR100 (GPCR142), and LGR7 (WO 2006/026355 and Changlu Liu et al., Mol Pharmacol. 67(1):231-40(2005)).

### [Chimeric Peptide of Human B-Chain and Human Relaxin-1 A-Chain]

B-chain: RAAPYGVRLCGREFIRAVIFTCGGSRW (SEQ ID NO: 5)
1 2

A-chain:   RPYVALFEKCCLIGCTKRSLAKYC (SEQ ID NO: 7)
                    31    3          2

### [Chimeric Peptide of Human B-Chain and Human Relaxin-2 A-Chain]

B-chain: RAAPYGVRLCGREFIRAVIFTCGGSRW (SEQ ID NO: 5)
1 2

A-chain:   QLYSALANKCCHVGCTKRSLARFC (SEQ ID NO: 8)
                    31    3          2

### [Chimeric Peptide of Human B-Chain and Modified A-Chain of Human Insulin-Like Peptide 3]

B-chain:   RAAPYGVRLCGREFIRAVIFTCGGSRW (SEQ ID NO: 5)
                    1                    2

A-chain: ATNPARYCCLSGCTQQDLLTLC (SEQ ID NO: 9)
31 3 2

### [Chimeric Peptide of Human B-Chain and Modified A-Chain of Human Insulin-Like Peptide 6]

B-chain: RAAPYGVRLCGREFIRAVIFTCGGSRW (SEQ ID NO: 5)
1 2

A-chain: GYSEKCCLTGCTKEELSIAC (SEQ ID NO: 10)
31 3 2

Relaxin-3 used in the present invention may be intramolecularly or intermolecularly bonded in the B-chain and A-chain through disulfide bonds or any other bonds, as long as it has substantially the same activities as those of relaxin-3. Examples of such peptides can be found typically in WO - 2004/113381; Halls et al., J. Pharmacol. Exp. Ther., 313, p.677-687,2005; Rosengren et al., J. Biol. Chem., 281, p.5845-5851,2006; and Bathgate et al., Biochemistry, 45, p.1043-1053,2006.

Relaxin-3, B-chain, A-chain, and the preproprotein used in the present invention can be obtained by various known methods, such as a genetic engineering method and a synthesis method. More specifically, in a genetic engineering method, a polynucleotide encoding relaxin-3, B-chain, A-chain, or the preproprotein is introduced into an appropriate host cell, the resulting transformant is cultured under the conditions for enabling the expression, and then the polypeptide of interest can be isolated and purified from the culture by a method generally used for isolation and purification of an expressed protein. In a synthesis method, synthesis can be carried out using an ordinary process such as a liquid phase process and a solid phase process. Generally an automatic synthesizer can be used. A chemically modified compound can be synthesized by an ordinary process.

### Polynucleotide Encoding Relaxin-3

A polynucleotide encoding relaxin-3, B-chain, A-chain, or the preproprotein used in the present invention (hereinafter also simply abbreviated as "polynucleotide encoding relaxin-3 used in the present invention") is not specifically limited, as long as it is a polynucleotide encoding relaxin-3, B-chain, A-chain, or the preproprotein used in the present invention. The term "polynucleotide" as used herein includes both DNA and RNA.

Examples of the polynucleotide encoding relaxin-3 used in the present invention includes a polynucleotide having the base sequence of the 76th (c) to the 156th (g) bases from the 5' end of SEQ ID NO: 1 (polynucleotide encoding human B-chain); a polynucleotide having the base sequence of the 355th (g) to the 426th (c) bases from the 5' end of SEQ ID NO: 1 (polynucleotide encoding human A-chain); and a polynucleotide which has a base sequence capable of hybridizing with a polynucleotide having the base sequence represented by SEQ ID NO: 1 (polynucleotide encoding human preproprotein) under stringent conditions and encodes a polypeptide having substantially the same activities as those of relaxin-3, B-chain, A-chain, or the preproprotein used in the present invention.

A specific example of the "polynucleotide which hybridizes under stringent conditions" in the present specification includes a polynucleotide having at least 70% or more, preferably 80% or more, more preferably 85% or more, further preferably 90% or more, further more preferably 95% or more, particularly preferably 98% or more, and most preferably 99% or more homology to a polynucleotide having the base sequence of the 76th (c) to the 156th (g) bases from the 5' end of SEQ ID NO: 1, a polynucleotide having the base sequence of the 355th (g) to the 426th (c) bases from the 5' end of SEQ ID NO: 1, or the base sequence represented by SEQ ID NO: 1, when the homology is calculated by a homology search software, such as FASTA, BLAST, Smith-Waterman (Meth. Enzym., 164, 765, 1988), using default parameters. Further, hybridization "under stringent conditions" can be performed, for example, by a method of carrying out the reaction at 40°C to 70°C, preferably at 60°C to 65°C, in a hybridization buffer solution generally used by those skilled in the art, and carrying out washing in a washing solution at a salt concentration of 15 to 300 mmol/L, preferably at 15 to 60 mmol/L. The temperature and salt concentration can be appropriately adjusted depending on the length of the probe to be used. The temperature and the salt concentration can be adjusted as appropriate according to the length of a probe to be used.

A polynucleotide encoding relaxin-3 used in the present invention can be, for example, of natural origin or entirely synthesized. Further, it can be synthesized using a part of a natural product. Typically, a polynucleotide encoding relaxin-3 used in the present invention can be obtained, for example, from a commercially available library or a cDNA library by a method customarily used in the field of genetic engineering, for example, by a screening method using an appropriate DNA probe constructed based on information of a partial amino acid sequence of relaxin-3, B-chain, A-chain, or the preproprotein used in the present invention.

An example of the polynucleotide encoding B-chain (polypeptide containing the amino acid sequence of the 26th (Arg) to the 52nd (Trp) residues from the N-terminus of SEQ ID NO: 2) includes a polynucleotide containing the base sequence of the 76th (c) to the 156th (g) bases from the 5' end of SEQ ID NO: 1.

An example of the polynucleotide encoding A-chain (polypeptide containing the amino acid sequence of the 119th (Asp) to the 142nd (Cys) residues from the N-terminus of SEQ ID NO: 2) includes a polynucleotide containing the base sequence of the 355th (g) to the 426th (c) bases from the 5' end of SEQ ID NO: 1.

An examples of the polynucleotide encoding the preproprotein (polypeptide containing the amino acid sequence represented by SEQ ID NO: 2) includes a polynucleotide containing the base sequence represented by SEQ ID NO: 1.

### Plasmid

A plasmid used in the transformation is not particularly limited, as long as it contains a polynucleotide encoding relaxin-3 used in the present invention. It can be obtained, for example, by inserting the polynucleotide into a known expression vector appropriately selected depending on a host cell used. It can also be a plasmid capable of expressing as a fused protein for cleaving relaxin-3, B-chain, A-chain, or the preproprotein used in the present invention, for easier operation in separation and purification.

### Transformant

The transformant is also not particularly limited, as long as it contains a polynucleotide encoding relaxin-3 used in the present invention. It can be, for example, a transformant in which the polynucleotide is incorporated into a chromosome of the host cell, a transformant which contains the polynucleotide in the form of a plasmid, or a transformant which does not express relaxin-3 used in the present invention. The transformant can be obtained, for example, by transforming a desired host cell with the plasmid or the polynucleotide itself. According to another embodiment, the transformant may further contain a plasmid capable of expressing a protease which acts on a cleavage site at which the B-chain and A-chain are cleaved.

Examples of the host cell include generally used known microorganisms such as Escherichia coli (e.g., E. coli JM109) and yeasts (e.g., Saccharomyces cerevisiae W303) and known cultured cells such as animal cells (e.g., CHO cells, HEK-293 cells, and COS cells) and insect cells (e.g., BmN4 cells).

Examples of the known expression vector include pUC, pTV, pGEX, pKK, and pTrcHis for E. coli; pEMBLY and pYES2 for yeasts; pcDNA3, pMAMneo, and pBabe Puro for CHO cells, HEK-293 cells, and COS cells; and a vector having the polyhedrin promoter of Bombyx mori nuclear polyhedrosis virus (BmNPV) (e.g., pBK283) for BmN4 cells.

The target polypeptide can be prepared by cultivating the transformant under such conditions that enable the expression of relaxin-3, B-chain, A-chain, or the preproprotein used in the present invention. Alternatively, it can be prepared by injecting RNA encoding relaxin-3, B-chain, A-chain, or the preproprotein used in the present invention into proper cells, and cultivating the cells under such conditions that enable the expression of reiaxin-3, B-chain, A-chain, or the preproprotein used in the present invention.

Relaxin-3, B-chain, A-chain, or the preproprotein used in the present invention can be obtained from a culture of the transformant, for example, by collecting microorganisms, cells, or cultured liquids, and obtaining the target through known separation and purification procedures in any combination while using the biochemical properties or physical properties of relaxin-3, B-chain, A-chain or the preproprotein. Usable techniques herein include ultrafiltration liquid chromatography such as affinity chromatography and high-performance liquid chromatography (HPLC); and dialysis techniques.

When the B-chain and A-chain of relaxin-3 used in the present invention are independently prepared, or when the B-chain or A-chain are prepared by cleaving from a fused protein, it is acceptable to isolate and purify the produced or cleaved B-chain and A-chain according to a common procedure, and to allow these chains to bond through disulfide bonds.

### Pharmaceutical Composition Containing Relaxin-3

### Definition

The term "mental condition" as used in the present specification means, for example, a condition of anxiety, tension, and/or depression.

The term "anxiety" as used in the present specification means an emotional condition or unpleasant emotional state indicated by a feeling such as fear or phobia accompanied by a physical sign such as sweating, tachycardia, accelerated breathing, or trembling. Anxiety is a normal feeling, but one with severe anxiety suffers from anxiety disorder. The "anxiety" therefore further means and includes anxiety disorders. Examples of the anxiety disorders include panic disorders with or without agoraphobia; agoraphobias without history of a panic disorder; specific phobias such as a phobia cued by a specific animal, or a social phobia; obsessive compulsive disorder; stress disorders including traumatic stress disorder and acute stress disorder; anxiety disorders induced by alcohol, drugs such as amphetamines, caffeine, cannabis, cocaine, hallucinogens, inhalants, and phencychdine, sedatives, hypnotics, and anxiolytics, and other substances; and anxiety disorders with anxiety or with anxiety in combination with depression. Such anxiety or anxiety disorders include those often related to other diseases such as mental diseases, immunological diseases, metabolic diseases, and gastrointestinal diseases, and other symptoms or include those induced by the other symptoms. The anxiety may occur with or without another disorder, such as depression in depressive disorders.

The term "depression" as used in the present specification means a feeling state of pessimistic distress, severe grief, disappointment, fluctuation, psychomotor retardation, diminished ability to concentrate, and self-deprecation. The "depression" further includes, at some levels, conditions inducing anorexia, weigh loss, hyperphagia, insomnia, hypersomnia, sexual impulse, and destruction of normal circadian rhythms in, for example, body temperature and endocrine functions.

Relaxin-3 and the polynucleotide encoding relaxin-3 used in the present invention can be used as antianxiety agents for treating mental conditions. They can be preferably used for treating anxiety. They can be used, for example, for treating disorders caused by certain abnormality in the regulation of mental conditions, and are preferably used for treating disorders caused by abnormality in the regulation of an anxiety activity. They can also be used for treating anxiety related to or induced by other diseases such as mental diseases, immunological diseases, metabolic diseases, and gastrointestinal diseases, and other symptoms; for treating anxiety disorders induced by alcohol, drugs, and other substances; and for treating and thereby mitigating anxiety upon examination or before or after surgery. They can also be used as medicines for treating diseases caused by abnormality in relaxin-3 or a polynucleotide encoding refaxin-3.

More specifically, relaxin-3 having an antianxiety activity has an activity of stabilizing mental conditions of humans or non-human organisms, because the anxiety activity is an unpleasant emotional condition and often accompanies physiological changes and behaviors resembling to those caused by fear. Consequently, relaxin-3 and a polypeptide encoding relaxin-3 can be used for treating anxiety disorders; generalized anxiety disorders; panic disorder; phobias; obsessive compulsive disorder; post traumatic stress disorder; treatment of post traumatic stress disorder; mental diseases such as depression, depressive symptoms, bipolar disorder, cyclothymia, affective disorder, emotional disturbance, sleep disorder, and schizophrenia; immunological diseases such as chronic rheumatoid arthritis, systemic lupus erythematosus, renal diseases, pachyderma, atopic dermatitis, bronchial asthma, multiple sclerosis, rheumatic pneumonitis, sarcoidosis, Crohn disease, inflammatory colitis, cirrhosis, chronic hepatitis, fulminant hepatitis, encephalomyelitis, and myasthenia gravis; metabolic diseases such as diabetes mellitus, obese diabetes, impaired glucose tolerance, ketosis, acidosis, diabetic neuropathy, diabetic nephropathia, diabetic retinopathy, hyperlipemia, arteriosclerosis, cardiac angina, myocardial infarction, obesity, adiposity, eating disorders, and anorexia nervosa; gastrointestinal diseases such as diarrhea, constipation, functional constipation, and irritable bowel syndrome; AIDS; cancer; and cachexia; anxiety related to or induced by the above diseases or symptoms; anxiety disorders induced by alcohol, drugs such as amphetamines, caffeine, cannabis, cocaine, hallucinogens, inhalants, and phencychdine, sedatives, hypnotics, and anxiolytics. In addition, they can be used for treating mental diseases accompanying anxiety symptoms, such as depression, depressive symptoms, bipolar disorder, cyclothymia, affective disorder, emotional disturbance, sleep disorders, and schizophrenia.

When used as a medicine for treating these diseases, relaxin-3 or polynucleotide encoding relaxin-3 used in the present invention can be used in the form of a salt, and in addition, they can also be used in the form of a hydrate. Such salts and hydrates are also included within the scope of the present invention. When used as a medicine for treating these diseases, a polynucleotide encoding relaxin-3 used in the present invention can be used alone or after being inserted into a proper vector, or after being added with a sequence such as signal sequence or polypeptide stabilizing sequence. Examples of the vector include known vectors such as adenovirus vector, retrovirus vector, Sendai virus (hemagglutinating virus of Japan) vector, plasmids, phagemids, and cosmids. Relaxin-3 or polynucleotide encoding relaxin-3 used in the present invention, a salt thereof, or a hydrate of them can be used alone or as a pharmaceutical composition by admixing with a pharmaceutically acceptable carrier.

The term "salt" as used herein is not particularly limited, as long as it is a salt formed with relaxin-3 or a polynucleotide encoding relaxin-3 used in the present invention and pharmaceutically acceptable. Preferred examples of such salts include hydrohalic acid salts such as hydrofluorides, hydrochlorides, hydrobromides, and hydroiodides; inorganic acid salts such as sulfates, nitrates, perchlorates, phosphates, carbonates, and hydrogen carbonates; organic carboxylates such as acetates, trifluroacetates, oxalates, maleates, tartrates, fumarates, and citrates; organic sulfonates such as methanesulfonates, trifluoromethanesulfonates, ethanesulfonates, benzensulfonates, toluenesulfonates, and camphorsulfonates; amino acid salts such as aspartates and glutamates; quaternary amine salts; alkaline metal salts such as sodium salts and potassium salts; and alkaline earth metal salts such as magnesium salts and calcium salts. More preferred examples as the "pharmaceutically acceptable salt" include trifuloroacetates, hydrochlorides, and oxalates.

The percentage of the active ingredient in the carrier can vary between 1 to 90 percent by weight. The medicine (agent) can be administered in various forms either orally or non-orally (for example, by intravenous, intramuscular, subcutaneous, rectal, or dermal administration) to humans or organisms other than humans. Examples of such other organisms than humans include non-human mammals such as cattle, monkeys, poultry, cats, mice, rats, hamsters, pigs, and canines; birds; reptiles; amphibians; fish; and insects. Accordingly, a pharmaceutical composition containing relaxin-3 or a polynucleotide encoding relaxin-3 according to the present invention is formulated into an appropriate dosage form depending on the administration route. Specifically, it can be formulated into oral formulations such as tablets, capsules, granules, dispersible powders, and syrups, or non-oral formulations such as injections, intravenous drips, liposome compositions, and suppositories. These pharmaceutical preparations can be manufactured by an ordinary process using commonly used excipients, fillers, binding agents, wetting agents, disintegrating agents, surfactants, lubricants, dispersing agents, buffering agents, preservatives, solubilizing agents, antiseptics, flavoring agents, analgesic agents, and stabilizers. Examples of the non-toxic additives to be used include lactose, fructose, glucose, starch, gelatin, magnesium stearate, methylcellulose or its salts, ethanol, citric acid, sodium chloride, and sodium phosphate.

The dosage form and amount of necessary dose depend on the selection of relaxin-3 or polynucleotide encoding relaxin-3 used in the present invention, the subject to be administered, the administration route, properties of the preparation, conditions of the patient, and physician's judgment. However, the appropriate dose per 1 kg of patient's body weight ranges, for example, from about 0.1 to 500 µg, preferably from about 0.1 to 100 µg, and more preferably from about 1 to 50 µg. The amount of necessary dose is expected to vary widely considering that the efficiency is different depending on the route of administration. For example, the necessary dose for oral administration is expected to be higher than that for intravenous injection. Such variations in the dose level can be adjusted using a standard empirical optimizing procedure well understood in the field.

### Method of Screening For Compounds Involved in Regulation of Mental Conditions Using Relaxin-3 Receptor

### Relaxin-3 Receptor

A relaxin-3 receptor used in the present invention can be, among various receptors, a receptor which has a binding ability to relaxin-3 used in the present invention and exhibits various cell-stimulating activities of the relaxin-3 receptor expressing cell (e.g., intracellular calcium release, adenylyl cyclase activation, intracellular cAMP production, intracellular cGMP production, inositol phospholipid production, electrical potential change in the cell membrane, pH change in the vicinity of the cell membrane, phosphorylation of intracellular proteins, c-fos and c-jun induction/activation, arachidonic acid release). The relaxin-3 receptor can be of any origin, as long as it satisfies the above conditions, and can be, for example, any of those derived from naturally occurring substances such as organs, tissues, and cells which express relaxin-3 receptors, of humans and non-human organisms including non-human mammals (e.g., mice, rats, hamsters, pigs, and canines), birds, reptiles, amphibians, fish, and insects; and those artificially prepared typically by a known genetic engineering technique or synthetic technique. A partial polypeptide of a relaxin-3 receptor used herein is not particularly limited, as long as it is usable in the after-mentioned screening method. It can be, for example, a partial polypeptide having a binding ability to relaxin-3 used in the present invention, or a partial polypeptide containing an amino acid sequence corresponding to the outside region of the cell membrane. The number of amino acids constituting the partial polypeptide herein is 90%, 80%, 70%, 60%, 50%, 40%, 30%, 20%, 10%, or 5% of the number of amino acids of the relaxin-3 receptor.

More specific examples usable as the relaxin-3 receptor include reported known receptors such as LGR7 (GenBank Accession No. NM_021634), SALPR (GenBank Accession No. NM_016568, also called GPCR135), and GPR100 (GenBank Accession No. AB_083593, also called hGPCR11 or GPCR142).

### Method of Screening for Compounds Involved in Regulation of Anxiety Action Using SALPR

The present invention will be illustrated in detail, with reference to a method of screening for compounds involved in the regulation of mental conditions, such as regulation of an anxiety activity (suppression or stimulation (acceleration) of an anxiety activity) using SALPR as an preferred embodiment of the present invention. Specifically, according to the present invention, there is provided a method of screening for a compound which has a binding ability to SALPR or a partial polypeptide thereof and is involved in the regulation of an anxiety activity (suppression or stimulation of an anxiety activity). In addition, whether or not a substance has an activity of suppressing or stimulating an anxiety activity can be determined by allowing the test substance to act on SALPR or a partial polypeptide thereof and measuring cell-stimulating activities.

SALPR or its partial polypeptide can be obtained by various known methods. It can be prepared, for example, by a known genetic engineering method using a polynucleotide encoding SALPR (GenBank Accession No. NM_D16568). In another embodiment, it can be obtained by a known polypeptide synthesis method, according to an ordinary procedure such as a liquid phase process or a solid phase process. An autosynthesizer can generally be used herein. Further, in another embodiment, a partial polypeptide of SALPR can be prepared by cleaving SALPR with an appropriate proteolytic enzyme. In yet another embodiment, it is desirable to prepare a partial polypeptide having a site with binding ability as the partial polypeptide of SALPR.

The polypeptide encoding SALPR used in the present invention means a polypeptide composed of the amino acid sequence represented by SEQ ID NO: 4, a modified polypeptide functionally equivalent to the polypeptide composed of the amino acid sequence represented by SEQ ID NO: 4, or a polypeptide which includes an amino acid sequence having 70% or more, preferably 80% or more, more preferably 85% or more, further preferably 90% or more, further more preferably 95% or more, particularly preferably 98% or more, and most preferably 99% or more, homology to the amino acid sequence represented by SEQ ID NO: 4 and exhibits substantially the same activities as those of SALPR (for example, a binding ability to relaxin-3 and various cell-stimulating activities associated with the binding, or regulation of an anxiety activity).

The modified polypeptide functionally equivalent to a polypeptide comprising the amino acid sequence represented by SEQ ID NO: 4 means a polypeptide in which one or more (preferably one or several) amino acids are deleted, substituted, inserted and/or added in the polypeptide comprising the amino acid sequence represented by SEQ ID NO: 4 and which exhibits substantially the same activities as those of SALPR (for example, a binding ability to relaxin-3 and various cell-stimulating activities associated with the binding, or regulation of an anxiety activity).

Further, a partial polypeptide of SALPR can also be used, as long as it has substantially the same activities as those of SALPR (for example, a binding ability to relaxin-3 and various cell-stimulating activities associated with the binding, or regulation of an anxiety activity). As the partial polypeptide of SALPR, a partial polypeptide having a site having a binding ability to relaxin-3 can be used.

The genetic engineering method will be explained in further detail using SALPR below; however, its partial peptide can also be used as long as it is usable in the screening method described later.

### Preparation of SALPR

A polynucleotide encoding SALPR is introduced into an appropriate host cell, the resulting transformant is cultured under the conditions for enabling the expression, then a polypeptide of interest can be obtained from the culture without purification or can be isolated and purified from the culture according to a procedure generally used for isolation and purification of an expressed protein, and thus SALPR is prepared. Examples of the procedure for the isolation and purification include ammonium sulphate salting-out, ion-exchange column chromatography using an ion-exchange cellulose, molecular sieving column chromatography using a molecular sieving gel, affinity column chromatography using a protein-A binding polysaccharide, dialysis, and lyophilization.

### Polynucleotide Encoding SALPR

A polynucleotide encoding SALPR used in the present invention is not particularly limited, as long as it is a polynucleotide encoding SALPR used in the present invention.

The term "polynucleotide" as used herein includes both DNA and RNA. More specifically, the polynucleotide used in the present invention is selected from the group consisting of the following polynucleotides (a) to (e):
(a) a polynucleotide including the base sequence represented by SEQ ID NO: 3;
(b) a polynucleotide encoding "a polypeptide consisting of the amino acid sequence represented by SEQ ID NO: 4";
(c) a polynucleotide encoding "a polypeptide which includes the amino acid sequence represented by SEQ ID NO: 4 and exhibits substantially the same activities as those of the SALPR";
(d) a polynucleotide encoding "a polypeptide which includes an amino acid sequence having deletions, substitutions, insertions and/or additions of one or more (preferably one or several) amino acids at one or more (preferably one or several) sites of the amino acid sequence represented by SEQ ID NO: 4 and exhibits substantially the same activities as those of the SALPR"; and
(e) a polynucleotide which hybridizes with a polynucleotide including the base sequence represented by SEQ ID NO: 3 under stringent conditions and encodes a polypeptide exhibiting substantially the same activities as those of the SALPR.

According to one embodiment of the present invention, the polynucleotide encoding SALPR used in the present invention is a polynucleotide including the base sequence represented by SEQ ID NO: 3. The polynucleotide represented by SEQ ID NO: 3 encodes SALPR including the amino acid sequence represented by SEQ ID NO: 4.

According to another embodiment of the present invention, the polynucleotide to be used in the present invention is a polynucleotide encoding "a polypeptide which includes an amino acid sequence having deletions, substitutions, insertion and/or additions of one or more (preferably one or several) amino acids at one or more (preferably one or several) sites of the amino acid sequence represented by SEQ ID NO: 4 and exhibits substantially the same activities as those of the SALPR." The number of amino acid residues which can be deleted, substituted, inserted and/or added is, for example, 1 to 30, preferably 1 to 20, more preferably 1 to 10, further more preferably 1 to 5, and most preferably 1 or 2.

According to still another embodiment of the present invention, the polynucleotide encoding SALPR used in the present invention is a polynucleotide "which hybridizes with a polynucleotide including the base sequence represented by SEQ ID NO: 3 under stringent conditions and encodes a polypeptide exhibiting substantially the same activities as those of the SALPR. Further, according to yet another embodiment of the present invention, the polynucleotide encoding SALPR used in the present invention is a polynucleotide "which hybridizes with a polynucleotide including the base sequence represented by SEQ ID NO: 3 under stringent conditions and encodes a polypeptide exhibiting substantially the same activities as those of the SALPR."

### Plasmid

A plasmid used in the above-mentioned transformation is not particularly limited, as long as it contains a polynucleotide encoding the SALPR. It can be obtained, for example, by inserting the polynucleotide into a known expression vector appropriately selected depending on a host cell used.

### Transformant

The transformant is also not particularly limited, as long as it contains a polynucleotide encoding the SALPR. It can be, for example, a transformant in which the polynucleotide is incorporated into a chromosome of a host cell, a transformant which contains the polynucleotide in the form of a plasmid, or a transformant which does not express SALPR. The transformant can be obtained, for example, by transforming a desired host cell with the plasmid or the polynucleotide itself.

Examples of the host cell include generally used known microorganisms such as Escherichia coli (e.g., E. coli JM109) and yeasts (e.g., Saccharomyces cerevisiae W303); and known cultured cells such as animal cells (e.g., CHO cells, HEK-293 cells, and COS cells) and insect cells (e.g., BmN4 cells).

Examples of the expression vector include pUC, pTV, pGEX, pKK, and pTrcHis for E. coli; pEMBLY and pYES2 for yeasts; pcDNA3, pMAMneo and pBabe Puro for CHO cells, HEK-293 cells, and COS cells; and a vector having the polyhedrin promoter of Bombyx mori nuclear polyhedrosis virus (BmNPV) (e.g., pBK283) for BmN4 cells.

A cell containing SALPR used herein is not particularly limited, as long as it expresses SALPR on the surface of the cell membrane. It can be obtained, for example, by culturing the transformant (namely, the cell transformed with a plasmid containing a polynucleotide encoding SALPR) under the conditions enabling the expression of SALPR, or by injecting RNA encoding SALPR into an appropriate cell and culturing it under the conditions enabling the expression of SALPR.

### Cell Membrane Fraction

A cell membrane fraction containing SALPR to be used in the present invention can be obtained, for example, by disrupting the cells expressing SALPR used in the present invention and then isolating a fraction rich in the cell membrane. Examples of the process of disrupting the cells include a process of crushing the cells using a homogenizer (e.g., a Potter-Elvehiem-type homogenizer), disruption by a Waring blender or Polytron (Kinematica), ultrasonic disruption, and disruption by ejecting the cells from a fine nozzle under pressure using a French press. Examples of the process for fractionating the cell membrane include a fractionation process by centrifugation, such as differential centrifugation and density gradient centrifugation.

SALPR, the cell membrane fraction (namely, a cell membrane fraction containing SALPR) or the cell (or the cell containing SALPR) can be used in a method of screening for a compound stimulating or suppressing an anxiety activity via SALPR according to the present invention.

Further, a screening method according to the present invention includes and utilizes, as the first embodiment, a method of examining whether a test substance binds specifically to SALPR, and, as the second embodiment, a method of examining cell-stimulating activities induced or generated by the binding of the test substance to SALPR (for example, intracellular calcium release, adenylyl cyclase activation, intracellular cAMP production, intracellular cGMP production, inositol phospholipid production, electrical potential change in the cell membrane, pH change in the vicinity of the cell membrane, phosphorylation of intracellular proteins, c-fos and c-jun induction/activation, and arachidonic acid release).

In the screening method according to the first embodiment of the present invention, for example, SALPR, the cell membrane fraction, or the cell is contacted with a test substance to analyze whether SALPR, the cell membrane fraction, or the cell binds to the test substance, and thus the screening for the compound can be achieved without distinction between anxiety activity-stimulating and suppressing abilities via SALPR.

Specifically, in the presence or absence of the test substance, SALPR, the membrane fraction or the cell is contacted with a labeled relaxin-3 to compare the amount of specific binding of relaxin-3 via SALPR, the cell membrane fraction, or the cell, and thus the screening for the compound can be achieved without distinction between anxiety activity-stimulating and suppressing abilities via SALPR. Namely, when the test substance has an anxiety activity-stimulating or suppressing ability via SALPR, the amount of specific binding of relaxin-3 via SALPR, the cell membrane fraction, or the cell in the presence of the test substance decreases as compared to the corresponding amount of the specific binding in the absence of the test substance.

A labeled relaxin-3 can be used as relaxin-3 so as to compare the amount of specific binding of relaxin-3 via SALPR, the cell membrane fraction, or the cell in the screening method according to the present invention. For the labeling, a radioactive isotope, an enzyme, a fluorescent substance, or a luminescent substance, for example, can be used. Examples of the radioactive isotope include [³H], [¹⁴C], [¹²⁵I], and [³⁵S]. Examples of the enzyme include β-galactosidase, alkaline phosphatase, and peroxidase. Examples of the fluorescent substance include fluorescein isothiocyanate and BODIPY. Examples of the luminescent substance include luciferin and lucigenin. Occasionally, the biotin-avidin system or an antibody against relaxin-3 can be used for binding of relaxin-3 with the labeling substance.

Thus, the screening method according to the present invention can screen for a compound which binds to SALPR, the cell membrane fraction, or the cell to inhibit their binding to relaxin-3 used in the present invention, without distinction between anxiety activity-stimulating and suppressing abilities via SALPR.

In the second embodiment of the screening method according to the present invention, the cell is contacted with a labeled relaxin-3 under conditions in the presence or absence of a test substance to compare the amount of specific binding of relaxin-3 via the cell under the conditions and then further compare a specific cell-stimulating activity of relaxin-3 under these conditions, thereby enabling the screening for a compound with distinction between anxiety activity-stimulating and suppressing abilities via SALPR.

In this embodiment, a substance which binds to the cell and exhibits the cell-stimulating activity via a receptor contained in the cell can be selected as a compound which suppresses an anxiety activity via SALPR.

On the other hand, in the embodiment, a test substance which inhibits binding of the cell and relaxin-3 but does not exhibit the cell-stimulating activity can be selected as a compound which stimulates or accelerates an anxiety activity via SALPR.

The screening method according to the present invention can be carried out using, for example, suppression of adenylyl cyclase activity as a cell-stimulating activity.

In the screening method according to this embodiment, for example, cAMP produced in a cell by the activation of adenylyl cyclase can be measured using a known method, thereby enabling the screening for a compound with distinction between anxiety activity-stimulating and suppressing abilities via SALPR. This embodiment utilizes intracellular signal transmission generated by the binding of relaxin-3 used in the present invention to SALPR, namely, the suppression of adenylyl cyclase activity which is one of cell-stimulating activities of SALPR. Specifically, when relaxin-3 binds to SALPR, a Gi family, a member of G protein family coupled with SALPR, suppresses adenylyl cyclase to decrease the amount of cyclic AMP (cAMP, produced from ATP by adenylyl cyclase) produced in the cell.

For example, the intracellular cAMP concentration increases when an adenylyl cyclase-activating agent [such as forskolin (FSK)] is added to mammal-derived cells (for example, HEK-293 cells or CHO cells) in which SALPR is expressed on the cell membrane (preferably, excessively expressed by introducing an expression vector containing SALPR).

Further, when relaxin-3 used in the present invention is added upon addition of an adenylyl cyclase-activating agent, adenylyl cyclase activity suppression also occurs due to the activity of relaxin-3 on SALPR used in the present invention, in addition to the adenylyl cyclase activity stimulation due to the adenylyl cyclase-activating agent, which results in a decrease in the cAMP production as compared to the case where the adenylyl cyclase-activating agent alone is added. Therefore, when the screening is carried out for a compound having an anxiety activity-suppressing activity, a compound which decreases the cAMP production (namely having the same activity as relaxin-3) can be selected by contacting the test substance alone, in place of relaxin-3 which acts via SALPR in this screening system.

When the screening is carried out for a compound having an anxiety activity-stimulating activity, an adenylyl cyclase-activating agent, relaxin-3 used in the present invention, and a test substance can be added to cells for screening. The cAMP production decreases due to the activity of relaxin-3 as compared to the case where the adenylyl cyclase-activating agent alone is added; however, the decrease in the cAMP production is suppressed when the test substance antagonizes the activity of relaxin-3. In this case, this test substance can be selected as a compound having an anxiety activity-stimulating activity.

An immunoassay, for example, can be used as a process for measuring the amount of intracellular cAMP. The measurement can also be carried out typically using a commercially available kit for cAMP quantification.

In another embodiment of the screening method, for example, screening for a compound can be achieved with distinction between anxiety activity-stimulating and suppressing abilities via SALPR, by using a cell (hereinafter also referred to as "screening cell") in which SALPR is expressed on the cell membrane (preferably excessively expressed by introducing an expression vector containing SALPR) and a reporter gene [for example, the alkaline phosphatase gene, the luciferase gene, the β-lactamase gene, the nitroreductase gene, the chloramphenicol acetyl transferase gene, the β-galactosidase gene, and a fluorescent protein gene such as GFP (green fluorescent protein) gene] having a cAMP responding element (CRE) located upstream of the 5' end is contained. Examples of. This embodiment utilizes the fact that the transcription of the reporter gene which has the CRE introduced into the above-mentioned screening cell, in the promoter region is suppressed as a result of the decrease in the cAMP production.

A process of screening for a compound with distinction between anxiety activity-stimulating and suppressing abilities via SALPR according to the embodiment above will be explained in more detail below.

Namely, the CRE introduced into the screening cell is a base sequence commonly present in a transcription regulatory region of a group of genes (cAMP inducing genes) whose expression is accelerated or stimulated when the intracellular cAMP concentration increases. Therefore, when an adenylyl cyclase-activating agent (e.g., FSK) is added to a screening cell, the intracellular cAMP concentration increases, which results in an increase in the amount of expression of the reporter gene located in the downstream of the CRE. The amount of expression of a reporter gene product can be easily measured by measuring luminescence obtainable from a luminescent substance generated from a substance reacted with the reporter gene product, or fluorescence obtainable from a fluorescent protein produced as the reporter gene product.

Further, when relaxin-3 used in the present invention is added upon addition of an adenylyl cyclase-activating agent, adenylyl cyclase activity suppression also occurs due to the activity of relaxin-3 on SALPR, in addition to the adenylyl cyclase activity stimulation due to the adenylyl cyclase-activating agent, which results in a decrease in the amount of the expression of the reporter gene product as compared to the case where the adenylyl cyclase-activating agent alone is added. Therefore, if the screening is for a compound having an anxiety activity-suppressing activity, a compound which decreases the amount of expression of the reporter gene product (namely having the same activity as relaxin-3) can be selected by contacting the test substance alone, in place of relaxin-3 which acts via SALPR in this screening system.

When the screening is carried out for a compound having an anxiety activity-stimulating activity, an adenylyl cyclase-activating agent, relaxin-3 used in the present invention, and a test substance can be added to a screening cell. The amount of expression of the reporter gene product decreases due to the activity of relaxin-3 as compared to the case where the adenylyl cyclase-activating agent alone is added; however, the decrease in the amount of expression of the reporter gene product is suppressed when the test substance antagonizes the activity of relaxin-3. In this case, the test substance can be selected as a compound having an anxiety activity-stimulating activity.

Whether the activity by a test substance is due to the activity through the binding to SALPR can be easily determined. For example, in parallel with the test using a screening cell (namely, a cell which expresses SALPR on the cell membrane and contains a reporter gene with CRE located upstream of the 5' end), a similar test is carried out using a control cell (for example, a cell which contains a reporter gene with CRE located upstream of the 5' end but does not express SALPR on the cell membrane). As a result, the screening cell and the control cell show the same phenomenon regarding the amount of expression of the reporter gene product when the activity by the test substance is not due to the binding to SALPR, while the screening cell and the control cell show different phenomena regarding the amount of expression of the reporter gene product when the activity by the test substance is due to the binding to SALPR.

In yet another embodiment, a test substance influencing anxiety activity regulation (namely, a compound which suppresses or stimulates an anxiety activity) can be detected and identified by administering the test substance, preferably one selected by the screening method, to humans or organisms other than humans [for example, non-human mammals (e.g., cattle, monkeys, poultry, cats, mice rats, hamsters, pigs, and canines), birds, reptiles, amphibians, fish, and insects] and measuring or observing after administration the variations in the behavior, amount of spontaneous motility, and parameters in the blood such as the amounts of hormones and secreted peptides in the blood or in the brain. Specifically, the behavior can be observed in a test such as a defensive burying test (Treit et al., Pharmacology Biochemistry and Behavior, 15, p.619-626, 1981), an open field test, a light/dark test, an elevated plus-maze test, a Geller-Seifter conflict test, Vogel conflict test, a social interaction test, a Hole-board test, a marble burying test, a fear conditioning stress test, a forced swimming test, or a tail suspension test. The non-human mammals are not limited to normal animals and further include animal models for genetic diseases and genetically modified animal models.

The test substance can be administered either orally or non-orally. Examples of the non-oral (parenteral) route include intravenous, intraarterial, subcutaneous, intraperitoneal, intratracheal, intrarectal, and intracerebral administrations, preferably administration into the cerebroventricle near the hypothalamus. A process for the administration of the test substance into the cerebroventricle of a test animal is not specifically limited, and can be carried out according to a common procedure to administer a medicine, for example, to a predetermined position in the cerebroventricle.

For example, a test animal is anaesthetized, and a guide cannula is fixed at a predetermined position by surgical operation. After elapse of an appropriate recuperative period (e.g., 7 days to 14 days, and preferably at least about 1 week), an injection needle is inserted into the guide cannula, and the test substance is administered via the needle using a microsyringe connected to a recycling pump. The dose of the test substance is not limited and can be set as appropriate. The test substance is generally prepared as a solution having a desired concentration typically using an artificial cerebrospinal fluid or physiological saline. The artificial cerebrospinal fluid is not limited and can be any of known commonly used artificial cerebrospinal fluids. A preferred example of the artificial cerebrospinal fluid includes aCSF (glucose 10 mM, KCl 2 mM, NaCl 115 mM, CaCl₂ 2.5 mM, MgSO₄ 1.2 mM, NaHCO₃ 25 mM, KH₂PO₄ 2,2 mM; pH 7.4). The test substance can be administered in a single or divided doses per day and the administration or observation period can be from one day to several weeks.

Relaxin-3 is preferably administered to a test animal by the same procedure as with the test substance. When cerebroventricularly administered to the test animal, relaxin-3 is preferably prepared as a solution having a desired concentration generally using an artificial cerebrospinal fluid, as with the test substance.

### Test Substance

The test substance herein can be any compound and can be, for example, an expression product of gene library, a synthetic low molecular-weight compound library, a nucleic acid (oligo DNA, oligo RNA), a synthetic peptide library, an antibody, a bacterially released substance, a fluid extract of cells (microorganisms, plant cells, or animal cells), a culture supernatant of cells (microorganisms, plant cells, or animal cells), a purified or partially purified polypeptide, an extract obtainable from a marine organism, plant or animal, soil, or a random phage peptide display library. The compound can be in the form of a salt, and the compound and a salt thereof can be in the form of a hydrate. These salts and hydrates are included in the test substance used in the present invention.

The term "salt" of a test compound as used herein refers to a pharmaceutically acceptable salt and is not particularly limited, as long as it is a pharmaceutically acceptable salt formed with the compound. Preferred examples of such salts include hydrohalic acid salts such as hydrofluorides, hydrochlorides, hydrobromides, and hydroiodides; inorganic acid salts such as sulfates, nitrates, perchlorates, phosphates, carbonates, hydrogen carbonates; organic carboxylates such as acetates, oxalates, maleates, tartrates, fumarates, and citrates; organic sulfonates such as methanesulfonates, trifluoromethanesulfonates, ethanesulfonates, benzensulfonates, toluenesulfonates, and camphorsulfonates; amino acid salts such as aspartates and glutamates; quaternary amine salts; alkaline metal salts such as sodium salts and potassium salts; and alkaline earth metal salts such as magnesium salts and calcium salts.

### Screening Kit

A screening kit according to an embodiment of the present invention contains at least a relaxin-3 receptor, the cell (namely, a cell containing a relaxin-3 receptor), or the cell membrane fraction (namely, a membrane fraction of a cell containing a relaxin-3 receptor). It may further occasionally contain relaxin-3. Relaxin-3 may be a labeled relaxin-3. The screening kit may further contain various reagents, such as a buffer solution for binding reaction, a buffer solution for washing, an instruction, and/or implements, if necessary. A preferred example of the relaxin-3 receptor used herein is SALPR.

A screening kit according to another embodiment of the present invention contains at least relaxin-3 used in the present invention, and a cell which expresses a relaxin-3 receptor on the cell membrane (preferably expresses excessively by introducing an expression vector containing relaxin-3 receptor) and moreover contains a reporter gene (e.g., alkaline phosphatase gene or luciferase gene) with a cAMP responding element (CRE) located upstream of the 5' end. The screening kit, if desired, may further contain various reagents such as a substrate for a reporter gene product (e.g., alkaline phosphatase or luciferase), an adenylyl cyclase-activating agent (e.g., FSK), a buffer solution for binding reaction, a buffer solution for washing, an instruction, and/or implements. The screening kit may further contain a cell which includes a reporter gene with a cAMP responding element (CRE) located upstream of the 5' end but does not express a relaxin-3 receptor on the cell membrane.

A preferred example of the relaxin-3 receptor used herein is SALPR.

### Pharmaceutical Composition Containing a Compound Obtained by Screening Method According to the Present Invention

A compound obtained by a screening method according to the present invention is a compound which is involved in the regulation of mental conditions, and preferably involved in the regulation of an anxiety activity (to stimulate or suppress an anxiety activity). The compound may be in the form of a salt. In addition, the compound and a salt thereof may be in the form of a hydrate.

Accordingly, a compound obtained by a method according to the present invention, a salt thereof, and a hydrate of them can be used for treating mental conditions. They can be preferably used as antianxiety agents for treating anxiety. They can be used, for example, in the treatment of disorders caused by certain abnormality in the regulation of mental conditions, preferably, an anxiety activity; the treatment of anxiety relating to other diseases and symptoms, such as mental diseases, immunological diseases, metabolic diseases, and gastrointestinal diseases, or anxiety induced by such other symptoms; the treatment of anxiety disorders induced by alcohol, drugs, and other substances; and the treatment and mitigation of anxiety upon examination or before or after surgery. They can also be used as medicines for treating diseases caused by abnormality in relaxin-3 or a polynucleotide encoding relaxin-3.

More specifically, since the anxiety activity is an unpleasant emotional condition and often accompanies physiological changes and behaviors resembling to those caused by fear, such a compound having an antianxiety activity has an activity of stabilizing mental conditions of humans or non-human organisms. Consequently, the compound, a salt thereof, and a hydrate of them can be used in the treatment of anxiety relating to or induced by diseases or symptoms including anxiety disorders; generalized anxiety disorders; panic disorder; phobias; obsessive compulsive disorder; post traumatic stress disorder; treatment of post traumatic stress disorder; mental diseases such as depression, depressive symptoms, bipolar disorder, cyclothymia, affective disorder, emotional disturbance, sleep disorder, and schizophrenia; immunological diseases such as chronic rheumatoid arthritis, systemic lupus erythematosus, renal diseases, pachyderma, atopic dermatitis, bronchial asthma, multiple sclerosis, rheumatic pneumonitis, sarcoidosis, Crohn disease, inflammatory colitis, cirrhosis, chronic hepatitis, fulminant hepatitis, encephalomyelitis, and myasthenia gravis; metabolic diseases such as diabetes mellitus, obese diabetes, impaired glucose tolerance, ketosis, acidosis, diabetic neuropathy, diabetic nephropathia, diabetic retinopathy, hyperlipemia, arteriosclerosis, cardiac angina, myocardial infarction, obesity, adiposity, eating disorders, and anorexia nervosa; gastrointestinal diseases such as diarrhea, constipation, functional constipation, and irritable bowel syndrome; AIDS; cancer; and cachexia; and anxiety disorders induced by alcohol, drugs such as amphetamines, caffeine, cannabis, cocaine, hallucinogens, inhalants, and phencychdine, sedatives, hypnotics, and anxiolytics. In addition, they can be used in the treatment of mental diseases accompanying anxiety symptoms, such as depression, depressive symptoms, bipolar disorder, cyclothymia, affective disorder, emotional disturbance, sleep disorders, and schizophrenia.

The compound obtained by a screening method according to the present invention, a salt thereof, or a hydrate of them can be used alone. However, it can also be used as a pharmaceutical composition by admixing with a pharmaceutically acceptable carrier. The percentage of the active ingredient in the carrier can vary in between 1 to 90 percent by weight. The medicament can be administered in various forms either orally or non-orally (for example, intravenous, intramuscular, subcutaneous, rectal, and dermal administrations) to humans or organisms other than humans [for example, non-human mammals (e.g., cattle, monkeys, poultry, cats, mice, rats, hamsters, pigs, and canines), birds, reptiles, amphibians, fish, and insects]. Accordingly, the pharmaceutical composition containing a compound obtained by a screening method according to the present invention, a salt thereof, or a hydrate of them is prepared into an appropriate form depending on the administration route. Specifically, it can be formulated into oral formulations such as tablets, capsules, granules, dispersible powders, and syrups, or non-oral formulations such as injections, intravenous drips, liposome compositions, and suppositories. These formulations can be manufactured by an ordinary process typically using commonly used excipients, fillers, binding agents, wetting agents, disintegrating agents, surfactants, lubricants, dispersing agents, buffering agents, preservatives, solubilizing agents, antiseptics, flavoring agents, analgesic agents, and stabilizers. Examples of the non-toxic additives used herein include lactose, fructose, glucose, starch, gelatin, magnesium stearate, methylcellulose or its salts, ethanol, citric acid, sodium chloride, and sodium phosphate.

The dosage form and amount of necessary dose depend on the selection of the compound obtained by the screening method according to the present invention, a salt thereof, or a hydrate of them, the subject to be administered, the administration route, properties of the preparation, conditions of the patient, and physician's judgment. However, the appropriate dose ranges, for example, from about 1.0 to 1500 µg, and preferably from about 10 to 500 µg, per 1 kg of patient's body weight. The amount of necessary dose is expected to vary widely considering that the efficiency is different depending on the route of administration. For example, the necessary dose for oral administration is expected to be higher than that for intravenous injection. Such variations in the dose level can be adjusted using a standard empirical optimizing procedure well understood in the field.

The term "treatment" as used herein generally means to obtain desired pharmacological effects and/or physiological effects. The effects are preventive in terms of completely or partly preventing diseases and/or symptoms or they are therapeutic in terms of completely or partly curing ill effects caused by diseases and/or symptoms. The term "treatment (therapy)" as used herein includes treatment of diseases in mammals, particularly humans, and are exemplified by the following treatments or therapies:
(a) to prevent the onset of a disease or symptoms in a patient who may have a causative factor for the disease or symptoms but is not diagnosed to have it;
(b) to inhibit disease symptoms, or to prevent or delay their progression; and
(c) to alleviate disease symptoms, that is, to regress a disease or symptoms or reverse the progression of the symptoms.

All of the prior art references cited in this specification are incorporated into the specification by reference.

### EXAMPLES

The present invention will be illustrated in further detail with reference to several examples below, which are by no means intended to limit the scope of the present invention.

### [Example 1] Preparation of Polynucleotide Encoding SALPR

Isolation of a polynucleotide encoding SALPR was carried out based on the nucleic acid sequence represented by SEQ ID NO: 3 as follows. In SEQ ID NO: 3, 1410 base pairs are shown and the area encoding SALPR is known to be from position 1 to position 1407 (1410 base pairs, 469 amino acid residues) (GenBank Accession No: NM_016568). To isolate a gene by a polymerase chain reaction (PCR), PCR primers represented by SEQ ID NO: 11 and SEQ ID NO: 12 were prepared according to an ordinary procedure.

Using a human genomic DNA (Roche Diagnostics) as a template, PCR was carried out with a set of PCR primers represented by SEQ ID NO: 11 and SEQ ID NO: 12 using the Expand High Fidelity PCR System (Roche Diagnostics) for 30 repeating cycles (at 98°C for 1 min, at 57°C for 1 min, and at 72°C for 3 min) according to the manufacture's instructions. As a result, an about 1400-base pair DNA fragment was obtained.

This DNA fragment was inserted into pCR2.1 (Invitrogen) and the sequence was confirmed by an ABI prism DNA sequencing kit (Perkin-Elmer Applied Biosystems). As a result, the sequence of 1410 base pairs, which was inserted into pCR2.1-SALPR obtained by the set of the primers consisting of SEQ ID NO: 11 and SEQ ID NO: 12, had a length the same as that from position 361 to position 1770 in SEQ ID NO: 3 but it had one mutation in the sequence. It is evident that this mutation does not influence the amino acid translated from the nucleic acid sequence at this site and thus a polynucleotide encoding SALPR was obtained.

### [Example 2] Preparation of Retrovirus Vector Plasmid

pBabe Puro (Morgenstern, J. P. and Land, H. Nucleic Acids Res. Vol. 18, 3587-3596 (1990) (SEQ ID NO: 13) was cleaved with SAlI and ClaI to remove the SV40 promoter-puro(r) region, and the resulting fragment was blunted with a Klenow fragment. Into the cleaved point the IRES-hyg(r) region, which had been excised from pIREShyg (Clontech) by cleaving with NsiI and XbaI and blunted with T4 polymerase, was inserted to obtain pBabeXIH.

pBabeXIH was cleaved with SspI and BamHI to remove the 5'-LTR-packaging signal. Into the cleaved point the 5'LTR-CMV promoter-packaging signal, which had been excised from pCLXSN (IMGENEX) by cleaving with SspI and BamHI, was inserted to obtain pBabeCLXIH.

### [Example 3] Preparation of Retrovirus Vector Plasmid for SALPR Gene Transfer

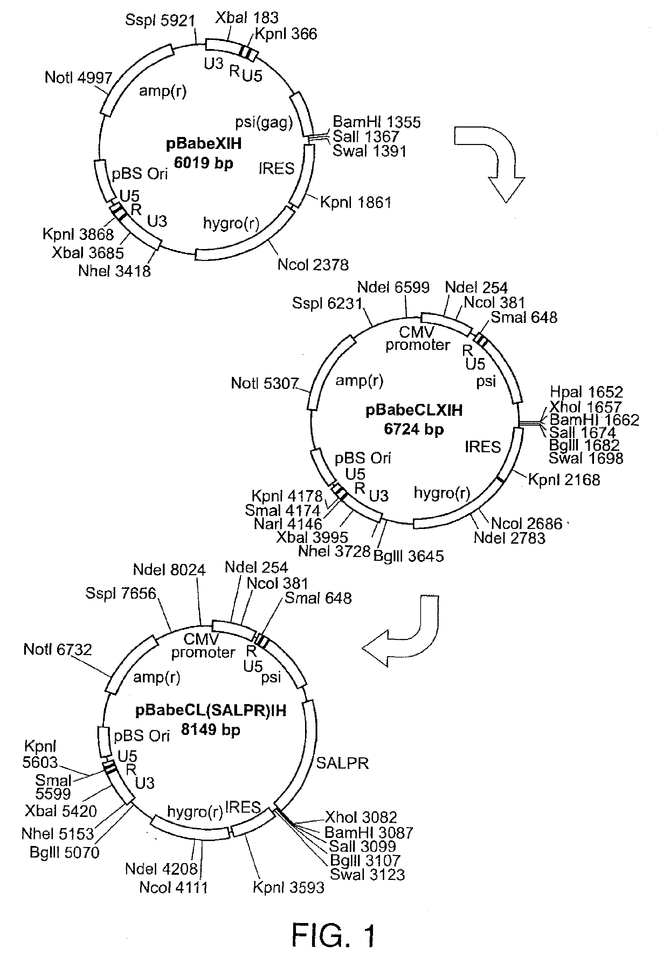

The retrovirus expression plasmid pBabeCLXIH described in Example 2 was cleaved with a restriction enzyme HpaI. Into the cleaved point a polynucleotide encoding SALPR, which had been excised from pCR2.1-SALPR obtained in Example 1 by cleaving with EcoRV and blunted with T4 polymerase, was inserted to obtain pBabeCL (SALPR) IH (Fig. 1).

### [Example 4] Preparation of Retrovirus Vector for SALPR Gene Transfer

In a 10-cm collagen-coated dish (IWAKI) were cultured 293-EBNA cells (Invitrogen) (2x 10⁶) using 10 ml of DMEM (Sigma) supplemented with 10% fetal bovine serum (FBS), 100 units/ml penicillin, and 100 µg/ml streptomycin (PS) (hereinafter referred to as "EBNA culture medium"). On the following day, the 293-EBNA cells were transfected using a lipofection reagent TransIT (Panvera) with 3.3 µg each of pV-gp (prepared by cleaving pVPack-GP (Stratagene) with NsiI and XbaI to remove IRES-hisD and blunting with T4 polymerase followed by selfligation of the resulting fragment), pVPack-VSV-G (Stratagene), and the retrovirus vector plasmid for SALPR gene transfer obtained in Example 3. The EBNA culture medium was exchanged 6 to 12 hours later and the incubation was continued at 37°C.

The culture solution was recovered 2 days after transfection and centrifuged at 1,200 x g for 10 minutes.

The resulting supernatant was filtered with a 0.45-µm filter (Millipore) to obtain an unconcentrated retrovirus vector fraction, and further concentration of the virus vector was carried out as follows.

50 Ultra-Clear Tubes (Beckman) for ultracentrifugation were sterilized with 70% ethanol and rinsed with distilled water, into which about 35 ml of the unconcentrated virus vector fraction was poured. The tubes were placed in an SW28 ultracentrifuge rotor (Beckman) and centrifuged at 19,500 rpm for 100 minutes using an XL-90 ultracentrifuge (Beckman). After centrifugation, the resulting supernatant was discarded and the tubes were kept in ice. One hour later, about 100 µl of a concentrated virus vector solution, i.e., the culture solution remaining on the tube wall, was obtained.

### [Example 5] Construction of SE302 Cell for Transferring Reporter Genes Containing a Cyclic AMP Responsive Element

### (1) Construction of Reporter DNA Containing a Cyclic AMP Responsive Element

A unit which involves in cAMP responsive transcription was constructed referring to a published paper (Durocher et al. Anal Biochem 2000, 284(2):316-26) as follows.

In order to construct a unit containing a cAMP responsive element (CRE), oligo DNAs represented by SEQ ID NO: 14 and SEQ ID NO: 15 for CREx2hb and oligo DNAs represented by SEQ ID NO: 16 and SEQ ID NO: 17 for CREx2bp were constructed according to an ordinary procedure.

The oligo DNAs of individual combinations were heat treated at 95°C, after which the temperature was gradually lowered to room temperature to form double-stranded DNAs (CREx2hb and CREx2bp). CREx2hb was digested with HindIII and BarrHI, and CREx2bp was digested with BamHI and PstI, and at the same time, pBfuescriptIISK(+) (Stratagene) was digested with HindIII and PstI. The digested DNAs were subjected to electrophoresis to purify DNAs having restriction enzyme cleavage sites on both ends, after which these three DNAs (CREx2hb, CREx2bp, and pBluescriptIISK(+)) were simultaneously ligated and the resulting plasmid sequences were analyzed to construct CRE4/pBluescriptIISK.

Next, in order to obtain DNA containing a VIP (vasoactive intestinal peptide) promoter, PCR primers represented by SEQ ID NO: 18 and SEQ ID NO: 19 were constructed according to an ordinary procedure.

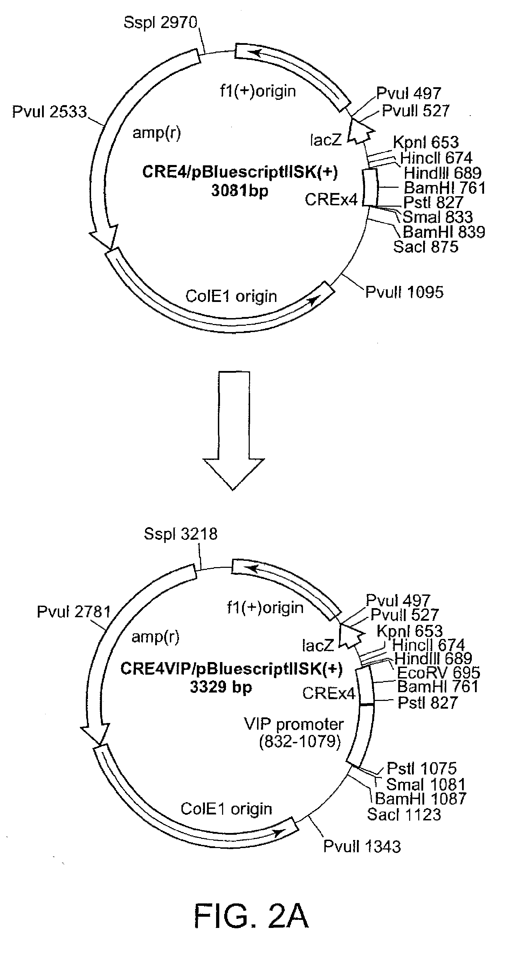

Using a human genomic DNA (Roche Diagnostics) as a template, PCR was carried out with a set of PCR primers represented by SEQ ID NO: 18 and SEQ ID NO: 19 using recombinant Taq polymerase (Takara) for 35 repeating cycles (at 94°C for 30 sec, at 55°C for 30 sec, and at 72°C for 1 min) to obtain a 264-base pair DNA fragment (SEQ ID NO: 20). This 264-base pair DNA was digested with PstI and inserted into the PstI site of CRE4/pBluescriptIISK(+), and the sequence of the resulting plasmid was confirmed to construct CRE4VIP/pBluescriptIISK(+) (Fig. 2A). CRE4VIP/pBluescriptIISK(+) thus obtained was digested with HindIII and SmaI, after which the resulting CRE4VIP promoter fragment was blunted.

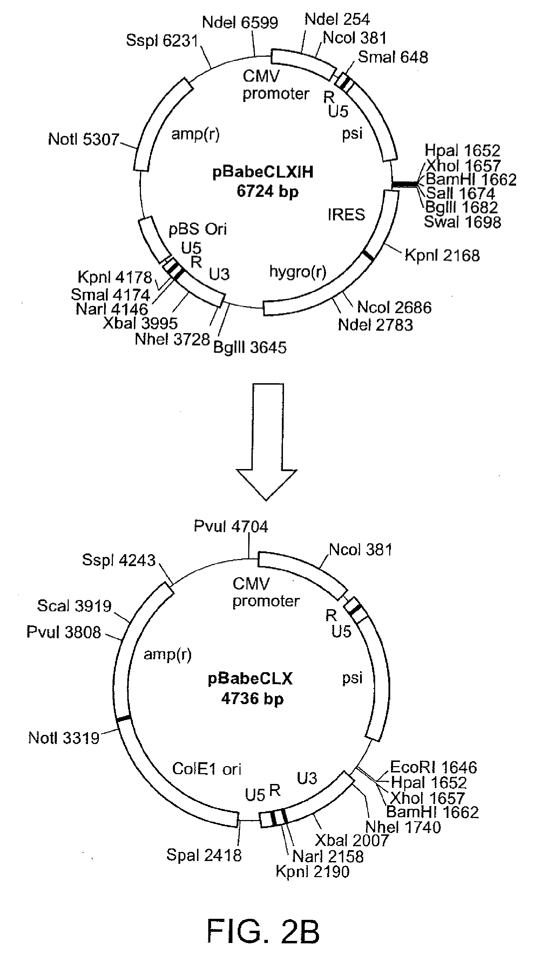

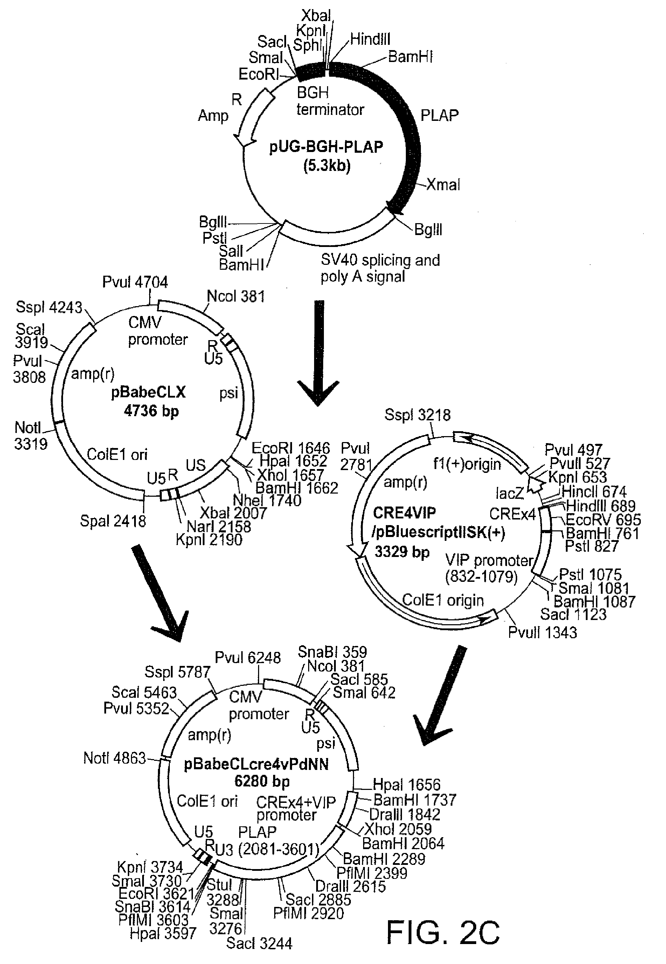

An IRES-hygro(r) region was removed from the above-mentioned viral expression vector plasmid pBabeCLXIH to construct pBabeCLX (Fig. 2B). A sequence containing CRE and a VIP promoter and a reporter gene, i.e., placenta-derived alkaline phosphatase (PLAP) gene (Goto et al., Molecular Pharmacology, 49, 860-873, 1996) were introduced into a retrovirus vector plasmid for foreign promoter transfer, which had been prepared by removing the NheI-NarI region in endogenous retrovirus enhancer activity (LTR) from pBabeCLX, to obtain pBabeCLcre4vPdNN (Fig. 2C).

### (2) Establishment of SE302 Cells for Transferring Reporter Genes Containing Cyclic AMP Responsive Element

A retrovirus vector was prepared according to the method described in Example 4 using a retrovirus vector plasmid pBabeCLcre4vPdNN in which the PLAP reporter gene is induced by a cyclic AMP responsive element. The retrovirus vector thus prepared was introduced into HEK293 cells and the resulting cells were cloned by the limiting dilution method. A cloned cell exhibiting best reactivity in PLAP induction (hereinafter called "SE302 cell") was used in the following experiments.

### [Example 6] Preparation of SALPR Expressing Cell Using Retrovirus Vector for SALPR Gene Transfer

SALPR gene transfer into a cell using the retrovirus vector prepared in Example 4 above was carried out as follows.

SE302 cells (3x 10³) constructed in Example 5 above were cultured in a 96-well plate (Asahi Techno Glass) using 100 µl of DMEM (Sigma) supplemented with 10% fetal bovine serum (FBS) and PS (hereinafter called "culture medium"). On the following day, the retrovirus vector prepared in Example 4 was appropriately diluted and a 100-µl portion of the dilution and polybrene (also called as hexadimethrine bromide, Sigma) prepared in the culture medium (at a final concentration of 8 µg/ml) were added to the SE302 cells. On the following day, the culture medium was replaced by 200 µl of a culture medium supplemented with 500 µg/ml hygromycin (Invitrogen) and then incubation was continued. The SE302 cells for SALPR gene transfer grown under these conditions (hereinafter called "SALPR-SE302 cells") were appropriately subcultured for experimental use.

### [Example 7] Suppression by Relaxin-3 of Transcription Activity Increased by Addition of Forskolin in SALPR-SE302 Cells

SALPR-SE302 cells constructed in Example 6 above were suspended in a medium for measuring transcription activity (DMEM supplemented with 10% FBS (inactivated at 65°C for 30 minutes)) and then inoculated in a 96-well plate (Beckton Dickinson) at 1x 10⁴ cells/well. On the following day, relaxin-3 (Phoenix Pharmaceuticals) or insulin (Invitrogen) diluted with an assay medium (DMEM supplemented with 0.1% bovine serum albumin) in specified concentrations was added, after which forskolin (Calbiochem) was added to make a final concentration of 1 µmol/L. After 1 day incubation, 15 µl each of the cell supernatant was recovered and then transferred to a 96-well plate for chemiluminescence measurement (Sumitomo Bakelite), 60 µl of buffer solution for assay (280 mmol/L Na₂CO₃-NaHCO₃, 8 mmol/L MgSO₄ pH 10) and 70 µl of Lumiphos530 (Lumigen) were added, and a reaction was carried out at room temperature for 1 hour, after which chemiluminescence for each well was measured by a fusion plate reader (Perkin Elmer) to assess the transcription activity. The activity in the cell supernatant added with each test sample was represented as a percent by setting the transcription activity in the cell supernatant with forskolin added at 1 µmol/L to be 100% and the activity in the supernatant without the addition of forskolin to be 0% (Fig. 3).

The result showed that relaxin-3 suppresses via SALPR activation the increase in transcription activity by forskolin. Since this increase in transcription activity was not affected by a related peptide, i.e., insulin, the reaction was revealed to be relaxin-3 specific. Namely, it was shown that compounds or substances which affect the activation of SALPR by relaxin-3 can be distinguished by using this experimental system.

### [Example 8] Antianxiety Activity by Intraventricular Administration of Relaxin-3 Using Defensive Burying Test (Rat)

The influence of relaxin-3 on an anxiety activity was determined using a defensive burying test (Treit et al., Pharmacology Biochemistry and Behavior, 15, p.619-626, 1981). The defensive burying test is an experimental system for evaluating anxiety activities and other mental symptoms such as depression state by using the phenomenon that, when a current stimulus is applied to a test animal via an electrode, the test animal shows a behavior to cover the electrode with a bedding material immediately after the shock.

Human relaxin-3 synthetically prepared in and supplied from Peptide Institute, Inc. (hereinafter also abbreviated as "human relaxin-3 (Peptide Institute, Inc.)") was used in the experiment. The human relaxin-3 is a polypeptide consisting of a polypeptide of the amino acid sequence of the 26th (Arg) to the 52nd (Trp) residues from the N-terminus of SEQ ID NO: 2 (human B-chain) and a polypeptide of the amino acid sequence of the 119th (Asp) to the 142nd (Cys) residues from the N-terminus of SEQ ID NO: 2 (human A-chain), wherein cysteine in B-chain at the 35th position from the N-terminus of SEQ ID NO: 2 is bonded to cysteine in A-chain at the 129th position from the N-terminus of SEQ ID NO: 2; cysteine in B-chain at the 47th position from the N-terminus of SEQ ID NO: 2 is bonded to cysteine in A-chain at the 142nd position from the N-terminus of SEQ ID NO: 2; and cysteine in A-chain at the 128th position from the N-terminus of SEQ ID NO: 2 is bonded to cysteine in A-chain at the 133rd position from the N-terminus of SEQ ID NO: 2.

### (1) Tested Rats and Pretreatment for Intracerebroventricular Administration

F344 male rats (7 weeks of age; Japan Charles River) were fed foods for experimental animals (MF; Oriental Yeast) to be adapted. The rats (150 to 200 g) received cannulation into the lateral cerebroventricle under anesthesia. Administration experiments of relaxin-3 were carried out a week or later.

### (2) Adaptation to Testing Chamber

The tested rats were placed and left in a testing chamber, which floor was covered with a bedding material to a height of 5 cm, for 30 minutes or more once a day from 3 days before the defensive burying test, so as to allow the tested rats to habituate to the testing environment. The rats were habituated to the environment without the stimulating electrode until the test.

### (3) Preparation of Relaxin-3 Solution

The human relaxin-3 (Peptide Institute, Inc.) was dissolved in physiological saline and was diluted to a final concentration of 0.05 nmol/rat or 1, nmol/rat, and thereby yielded a relaxin-3 solution.

### (4) Intracerebroventricular Administration of Relaxin-3 Solution

The tested rats with guide cannula implantation were divided into three groups and administered with 5 µL each of the human relaxin-3 administration solution (0.05 nmol/rat, N=6, 1 nmol/rat, N=8) or a vehicle solution (physiological saline, N=6) at a rate of 5 µl per 2 minutes using an infusion pump.

### (5) Implement of Defensive Burying Test and Observation of Behavior

On test day, the tested rats were placed in a test chamber (with bedding material to a height of 5 cm) with a stimulating electrode, so as to start an experiment. The tested rat received a 5 mA electrical shock when the rat touched the stimulating electrode. The 15 min (900 seconds) testing period began once the rat received its first shock and the electrode remained electrified at 5 mA for the remainder of the testing period. All behaviors of the tested rats were recorded using a videocamera, and the burying behavior time (burying time (sec.)) within 15 minutes from the first electrical shock was measured using the recorded tape. The burying behavior is defined as the behavior in which the experimental rat puts a bedding material toward the electrode with its fore paws. To compare among the human relaxin-3 administration groups and the vehicle group, a significant difference test using the Dunnett multiple comparison test procedure was conducted. In Fig. 4, the asterisk (*) means that P<0.05. With reference to Fig. 4, the burying behavior time was decreased in the groups which received human relaxin-3 (Peptide Institute, Inc.), and there was a significant decrease in the group of rats which received 1 nmol of human relaxin-3. These results revealed that relaxin-3 has an antianxiety activity.

### [Example 9] Antianxiety Activity by Relaxin-3 Intracerebroventricular Administration Using Elevated Plus-Maze (Mice)

The influence of relaxin-3 on anxiety activity was determined using an elevated plus-maze. An elevated plus-maze test is a behavioral pharmacological test which is widely used in the measurement of anxiety level of experimental animals such as rats or mice, or for the evaluation of drug efficacies of antianxiety drugs, using exploring behaviors in open arms, such as the time spent in open arms, as an index.

### (1) Tested Mice and Pretreatment for Intracerebroventricular Administration

BALB/c male mice (7 weeks of age; Japan Charles River) were received cannulation into the lateral cerebroventricle under anesthesia. The mice were then fed, and administration experiments of relaxin-3 were carried out a week or later.

### (2) Preparation of Relaxin-3 Solution

The human relaxin-3 (Peptide Institute, Inc.) was dissolved in physiological saline and was diluted to a final concentration of 1 nmol/mouse.

### (3) Intracerebroventricular Administration of Relaxin-3 Solution

The tested mice with guide cannula implantation were intracerebroventricularly administered with each 2 µL of the human relaxin-3 administration solution (N=8) or a vehicle solution (physiological saline) (N=9) at a rate of 1 µL per minute using an infusion pump.

### (4) Measurement of an Antianxiety Activity

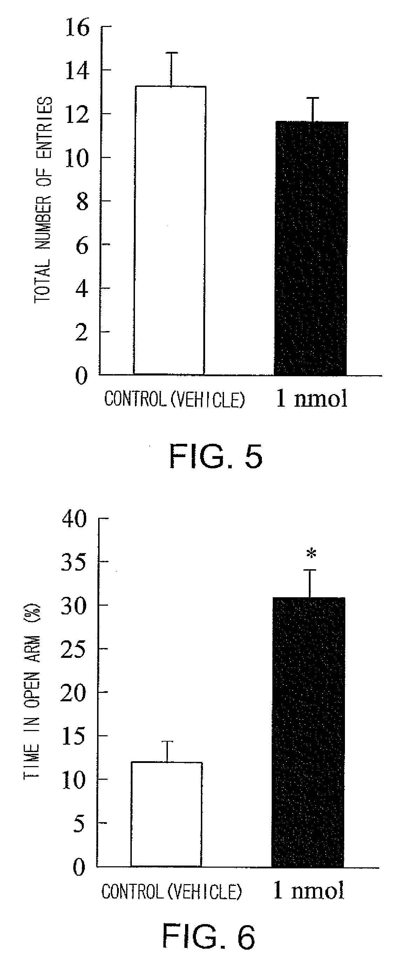

Antianxiety activities were measured using an elevated plus-maze. The maze was raised 45 cm above the floor and included four arms arranged in the form of a plus sign (+) spread from the central platform (5 cm x 5 cm) positioned at the center. Two opposite arms (30 cm long and 5 cm wide) are open arms, and the other two opposite arms (30 cm long and 5 cm wide with walls 15 cm high) were closed arms. Lighting was arranged so as to set the luminance at the floor in the open arms was 60 to 80 lux. A mouse after 10 minutes of the intracerebroventricular administration was placed in the center of the plus-maze so as to face one of the open arms, and the test was carried out for 5 minutes. The test was conducted only within a period from 11:00 to 16:00. The behaviors of mice were recorded by a video, and the number of entries into the open and closed arms and the time spent in the open and closed arms were measured on each mouse by analyzing the video after the completion of the test. Figs. 5 and 6 show the total number of entries into open and closed arms and the percentage of time spent in open arms within the testing period of 5 minutes, respectively. To compare between the human relaxin-3 administration group and the vehicle group, a significant difference test by the t-test was conducted. In Figs. 5 and 6, the asterisk (*) indicates that P<0.05. Fig. 5 demonstrates that there was no difference in the total number of entries into open and closed arms between the human relaxin-3 administration group and the vehicle group. However, Fig. 6 demonstrates that there was a significant increase in the time spent in open arms of the human relaxin-3 administration group as compared with the vehicle group. These results showed that relaxin-3 has an antianxiety activity.

### [Example 10] Antianxiety Activity by Relaxin-3 Intracerebroventricular Administration Assayed Using Elevated Plus-Maze (Rats)

The influence of relaxin-3 on anxiety activity was determined using an elevated plus-maze.

### (1) Tested Rats and Pretreatment for IntraCerebraventricular Administration

Wistar male rats (9 weeks of age; Japan Charles River) were received cannulation into the lateral cerebroventricle under anesthesia. The rats were then fed, and administration experiments of relaxin-3 were carried out a week or later.

### (2) Preparation of Relaxin-3 Solution

The human relaxin-3 (Peptide Institute, Inc.) was dissolved in physiological saline and was diluted to a final concentration of 10 pmol/rat or 50 pmol/rat.

### (3) Intracerebroventricular Administration of Relaxin-3 Solution

The tested rats with guide cannula implantation were administered with 5 µL each of the human relaxin-3 administration solution (10 pmol/rat, N=8; or 50 pmol/rat, N=7), or vehicle group (physiological saline, N=7) at a rate of 2.5 µL per minute using an infusion pump.

### (4) Measurement of an Antianxiety Activity

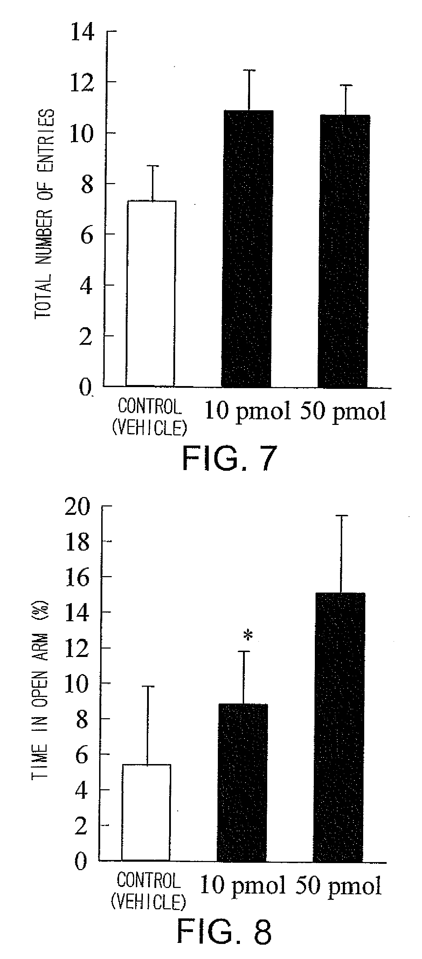

Antianxiety activities were measured using an elevated plus-maze. The maze was raised 50 cm above the floor and included four arms arranged in the form of a plus sign (+) spread from the central platform (10 cm x 10 cm) positioned at the center. Two opposite arms (50 cm long and 10 cm wide) are open arms, and the other two opposite arms (50 cm long, 10 cm wide with walls 40 cm high) were closed arms. Lighting was arranged so as to set the luminance at the floor in the open arms was 60 to 80 lux. A rat 10 minutes after the intracerebroventricular administration was placed in the center of the plus-maze so as to face one of the open arms, and the test was carried out for 5 minutes. The test was conducted only within a period from 11:00 to 16:00. The behaviors of rats were recorded by a video, and the number of entries into the open and closed arms and the time spent in the open and closed arms were measured on each mouse by analyzing the video after the completion of the test. Figs. 7 and 8 show the total number of entries into open and closed arms and the percentage of time spent in open arms within 5 minutes, respectively. To compare among the human relaxin-3 administration groups and the vehicle group, a significant difference test by the t-test was conducted. In Figs. 7 and 8, the asterisk (*) indicates that P<0.05. Fig. 7 demonstrates that there was no difference in the total number of entries into open and closed arms between the 10 pmol/rat and 50 pmol/rat administration groups and the vehicle group. However, Fig. 8 demonstrates that there was an increase in the time spent in open arms of the 10 pmol/rat and 50 pmol/rat administration groups as compared with the vehicle group; and the activity in the 10 pmol/rat group is statistically significantly high. These results showed that relaxin-3 has an antianxiety activity.

### Industrial Applicability

Relaxin-3 has an antianxiety activity and is thereby useful typically in the treatment of anxiety. A compound capable of activating or suppressing a relaxin-3 receptor, a salt thereof, or a hydrate of them is usable in the therapy of anxiety, because relaxin-3 has an antianxiety activity. Accordingly, a method of screening for a compound which is involved in the regulation of anxiety and activates or suppresses a relaxin-3 receptor, a salt thereof, or a hydrate of them, as well as a screening kit used in the screening method, are useful.

## Claims

1. An antianxiety agent, comprising relaxin-3, a salt thereof, or a hydrate of them.

2. The antianxiety agent according to claim 1, wherein relaxin-3 is human relaxin-3.

3. The antianxiety agent according to claim 1, wherein relaxin-3 is a polypeptide consisting of an A-chain and a B-chain which are obtainable from a functionally equivalent modified polypeptide of a relaxin-3 preproprotein, or consisting of an A-chain and a B-chain which are obtainable from a homologous polypeptide of a relaxin-3 preproprotein, and wherein cysteine residues of the A-chain and the B-chain are bonded through disulfide bonds.

4. A method of screening for a compound having an antianxiety activity, a salt thereof, or a hydrate of them, the method comprising the steps of:
(A) contacting a test substance with a relaxin-3 receptor, a cell containing a relaxin-3 receptor, or a membrane fraction of the cell; and
(B) measuring a cell-stimulating activity via the relaxin-3 receptor.

5. A method of screening for a compound suppressing or stimulating an anxiety activity, a salt thereof, or a hydrate of them, the method comprising the step of:
(A) contacting a test substance and relaxin-3, a salt thereof, or a hydrate of them with a relaxin-3 receptor, a cell containing a relaxin-3 receptor, or a membrane fraction of the cell.

6. The method of screening according to claim 5, wherein relaxin-3 is human relaxin-3.

7. The method of screening according to claim 5, wherein relaxin-3 is a polypeptide consisting of an A-chain and a B-chain which are obtainable from a functionally equivalent modified polypeptide of a relaxin-3 preproprotein, or consisting of an A-chain and a B-chain which are obtainable from a homologous polypeptide of a relaxin-3 preproprotein, and wherein cysteine residues of the A-chain and the B-chain are bonded through disulfide bonds.

8. The method of screening for a compound suppressing or stimulating an anxiety activity, a salt thereof, or a hydrate of them according to any one of claims 5 to 7, further comprising the step of:
(B) measuring a cell-stimulating activity via the relaxin-3 receptor.

9. The method of screening according to any one of claims 4 to 8, wherein the relaxin-3 receptor is a somatostatin- and angiogenin-like peptide receptor (SALPR) or a partial polypeptide thereof.

10. The method of screening according to claim 9, wherein the SALPR is a polypeptide comprising the amino acid sequence represented by SEQ ID NO: 4.

11. A kit for screening for a compound having an antianxiety activity, a salt thereof, or a hydrate of them, comprising relaxin-3 receptor, a cell containing a relaxin-3 receptor, or a membrane fraction of the cell.

12. The kit for screening according to claim 11, further comprising relaxin-3, a salt thereof, or a hydrate of them.

13. The kit for screening according to claim 12, wherein relaxin-3 is human relaxin-3.

14. The kit for screening according to claim 12, wherein relaxin-3 is a polypeptide consisting of an A-chain and a B-chain which are obtainable from a functionally equivalent modified polypeptide of a relaxin-3 preproprotein, or consisting of an A-chain and a B-chain which are obtainable from a homologous polypeptide of a relaxin-3 preproprotein, and wherein cysteine residues of the A-chain and the B-chain are bonded through disulfide bonds.

15. The kit for screening according to any one of claims 12 to 14, wherein relaxin-3 is labeled.

16. The kit for screening according to any one of claims 11 to 15, wherein the relaxin-3 receptor is a SALPR or a partial polypeptide thereof.

17. The kit for screening according to claim 16, wherein the SALPR is a polypeptide comprising the amino acid sequence represented by SEQ ID NO: 4.

18. A method of screening for a compound suppressing or stimulating an anxiety activity, a salt thereof, or a hydrate of them, the method comprising the steps of administering a compound acting on a relaxin-3 receptor to a human or a non-human organism; and measuring an anxiety activity after administration.

19. The method of screening according to claim 18, wherein the step of measuring an anxiety activity comprises carrying out a defensive burying test or an elevated plus-maze test.

20. The method of screening according to one of claims 18 and 19, wherein the compound acting on a relaxin-3 receptor is a compound obtained through the method of any one of claims 4 to 10.
